# EUROPÄISCHE PATENTSCHRIFT

(11) **EP 2 344 116 B1**
(45) Veröffentlichungstag und Bekanntmachung des Hinweises auf die Patenterteilung: **05.10.2016**
(21) Anmeldenummer: 09781418.0
(22) Anmeldetag: 03.08.2009
(51) Int. Cl.: A61K 8/64, A61Q 5/12, A61Q 19/00, A61Q 5/00, A61Q 5/02

(54) **HAARBEHANDLUNGSMITTEL MIT NIEDRIGDOSIERTEN OLIGOPEPTIDEN**
HAIR TREATMENT AGENT HAVING LOW-DOSE OLIGOPEPTIDES
AGENTS DE TRAITEMENT CAPILLAIRE COMPRENANT DES OLIGOPEPTIDES FAIBLEMENT DOSÉS

(30) Priorität: 03.09.2008 DE 102008045511
(43) Veröffentlichungstag der Anmeldung: 20.07.2011
(73) Patentinhaber: Henkel AG & Co. KGaA, 40589 Düsseldorf (DE)
(72) Erfinder: SCHULZE ZUR WIESCHE, Erik, 20144 Hamburg (DE); WESTPHAL, Petra, 21629 Neu Wulmstorf (DE); POPPE, Elisabeth, 22763 Hamburg (DE)
(86) Internationale Anmeldenummer: PCT/EP2009/060026
(87) Internationale Veröffentlichungsnummer: WO 2010/026010

(56) Entgegenhaltungen:
- EP-A2- 1 310 511
- WO-A1-2009/010314
- DE-A1- 19 540 853
- DE-A1-102007 039 743
- US-A1- 2002 035 243

## Beschreibung

Die Erfindung betrifft Haarbehandlungsmittel bestimmte Oligopeptide sowie die Verwendung dieser Mittel zur Reinigung und/oder Pflege von Haut und Haar.

Nicht zuletzt durch die starke Beanspruchung der Haare, beispielsweise durch das Färben oder Dauerwellen als auch durch die Reinigung der Haare mit Shampoos und durch Umweltbelastungen, nimmt die Bedeutung von Pflegeprodukten mit möglichst langanhaltender Wirkung zu. Derartige Pflegemittel beeinflussen die natürliche Struktur und die Eigenschaften der Haare. So können anschließend an solche Behandlungen beispielsweise die Naß- und Trockenkämmbarkeit des Haares, der Halt und die Fülle des Haares optimiert sein oder die Haare vor erhöhtem Spliß geschützt sein.
Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung die Kämmbarkeit, der Halt und die Fülle der Haare verbessert und die Splißrate verringert.
Die bekannten Wirkstoffe können jedoch nicht alle Bedürfnisse in ausreichendem Maße abdecken. Es besteht daher weiterhin ein Bedarf nach Wirkstoffen bzw. Wirkstoffkombinationen für kosmetische Mittel mit guten pflegenden Eigenschaften und guter biologischer Abbaubarkeit. Insbesondere in farbstoff- und/oder elektrolythaltigen Formulierungen besteht Bedarf an zusätzlichen pflegenden Wirkstoffen, die sich problemlos in bekannte Formulierungen einarbeiten lassen und dort nicht aufgrund von Unverträglichkeiten mit anderen Inhaltsstoffen in ihrer Wirkung abgeschwächt werden.
Es wurde nun gefunden, daß besonders vorteilhafte Ergebnisse erzielt werden, wenn man bestimmte Oligopeptide in Haarbehandlungsmittel inkorporiert.

Die EP 1 310 511 A1 offenbart Haarwachstumsmittel, welche ein Oligopeptid mit der Sequenz Pro-Ser-Glu-Gln-Ser-Cys-Ala-Glu-Glu-Glu enthalten.

Die DE 195 40 853 A1 offenbart Haarbehandlungsmittel, welche Proteinhydrolysate (Oligopeptide) enthalten. Bestimmte Aminosäuresequenzen werden hier nicht offenbart.

Ein erster Gegenstand der vorliegenden Erfindung sind Haarbehandlungsmittel, enthaltend 0,00001 bis < 0,05 Gew.-% mindestens eines Oligopetids, das mindestens eine Aminosäuresequenz Tyr-Glu-Glu 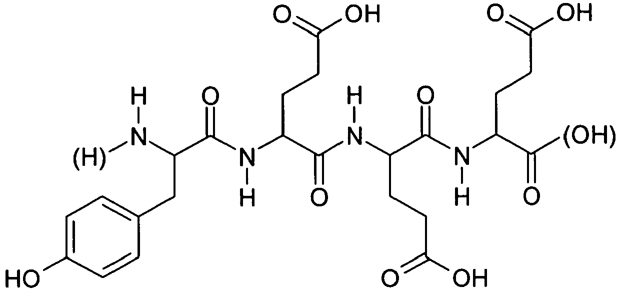 aufweist, wobei die Amino-Gruppe frei oder protoniert und die Carboxy-Gruppen frei oder deprotoniert vorliegen können, mit der Maßgabe, daß die Gesamtmenge an Oligopeptiden, die eine Aminosäuresequenz Glu-Glu-Glu aufweisen, 0,00001 bis < 0,05 Gew.-% beträgt In dieser wie in allen nachstehenden Formeln bedeutet das eingeklammerte Wasserstoffatom der Aminogruppe ebenso wie die eingeklammerte Hydroxygruppe der Säurefunktion, daß die betreffenden Gruppen als solche vorhanden sein können (dann handelt es sich um ein Oligopeptid mit der betreffenden Anzahl an Aminosäuren wie dargestellt (in der vorstehenden Formel 4) oder aber, daß die Aminosäuresequenz in einem Oligopeptid vorliegt, das noch weitere Anminosäuren umfaßt - je nachdem, wo die weitere(n) Aminosäure(n) gebunden ist/sind, sind die eingeklammerten Bestandteile der o.g. Formel durch den/die weiteren Aminosäurerest(e) ersetzt.
Haarbehandlungsmittel im Sinne der vorliegenden Erfindung sind beispielsweise Haarshampoos, Haarkonditionierer, konditionierenden Shampoos, Haarsprays, Haarspülungen, Haarkuren, Haarpackungen, Haar-Tonics, Dauerwell-Fixierlösungen, Haarfärbeshampoos, Haarfärbemittel, Haarfestiger, Haarlegemittel, Haarstyling-Zubereitungen, Fönwell-Lotionen, Schaumfestiger, Haargele, Haarwachse oder deren Kombinationen. Im Hinblick auf die Tatsache, daß Männer oft die Anwendung mehrerer unterschiedlicher Mittel und/oder mehrere Anwendungsschritte scheuen, sind erfindungsgemäße Mittel bevorzugt solche Mittel, die der Mann ohnehin anwendet. Bevorzugte erfindungsgemäße Mittel sind daher Shampoos, Konditioniermittel oder Haar-Tonics. Die erfindungsgemäßen Haarbehandlungsmittel enthalten - bezogen auf ihr Gewicht - 0,00001 bis < 0,05 Gew.% mindestens eines Oligopeptids, das mindestens eine Aminosäuresequent Tyr-Glu-Glu-Glu, d.h. mindestens drei aufeinander folgende Glutaminsäuren aufweist. Oligopeptide im Sinne der vorliegenden Anmeldung sind durch Peptid-Bindungen Säureamidartig verknüpfte Kondensationsprodukte von Aminosäuren, die mindestens 4 und maximal 25 Aminosäuren umfassen
In erfindungsgemäß bevorzugten Haarbehandlungsmitteln umfaßt das Oligopeptid 5 bis 15 Aminosäuren, vorzugsweise 6 bis 13 Aminosäuren, besonders bevorzugt 7 bis 12 Aminosäuren und insbesondere 8, 9 oder 10 Aminosäuren.
Je nachdem, ob weitere Aminosäuren an die Sequenz Tyr-Glu-Glu-Glu gebunden sind und je nach Art dieser Aminosäuren kann die Molmasse des in den erfindungsgemäßen Mitteln enthaltenen Oligopeptids variieren. Erfindungsgemäß bevorzugte Haarbehandlungsmittel sind dadurch gekennzeichnet, daß das Oligopeptid eine Molmasse von 650 bis 3000 Da, vorzugsweise von 750 bis 2500 Da, besonders bevorzugt von 850 bis 2000 Da und insbesondere von 1000 bis 1600 Da aufweist.
Wie aus der bevorzugten Anzahl von Aminisäuren in den Oligopeptiden und dem bevorzugten Molmassenbereich zu ersehen ist, werden vorzugsweise Oligopeptide eingesetzt, die nicht allein aus der Sequenz Tyr-Glu-Glu-Glu bestehen, sondern weitere, an diese Sequenz gebundene Aminosäuren aufweisen. Diese weiteren Aminosäuren sind vorzugsweise aus bestimmten Aminosäuren ausgewählt, während bestimmte andere Vertreter erfindungsgemäß weniger bevorzugt sind. So ist es bevorzugt, wenn die in den erfindungsgemäßen Mitteln eingesetzten Oligopeptide kein Methionin enthalten.
Weiter bevorzugt ist es, wenn die in den erfindungsgemäßen Mitteln eingesetzten Oligopeptide kein Cystein und/oder Cystin enthalten.
Weiter bevorzugt ist es, wenn die in den erfindungsgemäßen Mitteln eingesetzten Oligopeptide keine Asparaginsäure und/oder Asparagin enthalten.
Weiter bevorzugt ist es, wenn die in den erfindungsgemäßen Mitteln eingesetzten Oligopeptide kein Serin und/oder Threonin enthalten.
Demgegenüber ist es bevorzugt, wenn die in den erfindungsgemäßen Mitteln eingesetzten Oligopeptide Tyrosin enthalten.
Weiter bevorzugt ist es, wenn die in den erfindungsgemäßen Mitteln eingesetzten Oligopeptide Leucin enthalten.
Weiter bevorzugt ist es, wenn die in den erfindungsgemäßen Mitteln eingesetzten Oligopeptide Isoleucin enthalten.
Weiter bevorzugt ist es, wenn die in den erfindungsgemäßen Mitteln eingesetzten Oligopeptide Arginin enthalten.
Weiter bevorzugt ist es, wenn die in den erfindungsgemäßen Mitteln eingesetzten Oligopeptide Valin enthalten.
Besonders bevorzugte Oligopeptide bzw in den bevorzugten Oligopeptiden enthaltene Aminosäuresequenzen werden nachstehend beschrieben:
Oligopeptide, die Tyrosin und Isoleucin aufweisen, sind erfindungsgemäß bevorzugt. Besonders bevorzugt sind dabei erfindungsgemäße Haarbehandlungsmittel, bei denen das in ihnen enthaltene Oligopeptid mindestens eine Aminosäuresequenz Tyr-Glu-Glu-Glu-Ile aufweist, wobei die Amino-Gruppe frei oder protoniert und die Carboxy-Gruppen frei oder deprotoniert vorliegen können.

Weiter bevorzugte Oligopeptide enthalten zusätzlich Arginin, das vorzugsweise an Isoleucin gebunden vorliegt deprotoniert vorliegen können.
Noch weiter bevorzugte Oligopeptide enthalten zusätzlich Valin, das vorzugsweise an das Arginin gebunden vorliegt. Erfindungsgemäß weiter bevorzugte Haarbehandlungsmittel sind daher dadurch gekennzeichnet, daß das in ihnen enthaltene Oligopeptid mindestens eine Aminosäuresequenz Tyr-Glu-Glu-Glu-Ile-Arg-Val aufweist, wobei die Amino-Gruppen frei oder protoniert und die Carboxy-Gruppen frei oder deprotoniert vorliegen können.
Noch weiter bevorzugte Oligopeptide enthalten zusätzlich Leucin, das vorzugsweise an das Valin gebunden vorliegt. Erfindungsgemäß weiter bevorzugte Haarbehandlungsmittel sind dadurch gekennzeichnet, daß das in ihnen enthaltene Oligopeptid mindestens eine Aminosäuresequenz Tyr-Glu-Glu-Glu-Ile-Arg-Val-Leu aufweist, wobei die Amino-Gruppen frei oder protoniert und die Carboxy-Gruppen frei oder deprotoniert vorliegen können.
Insbesondere bevorzugte Oligopeptide enthalten zusätzlich Leucin, das vorzugsweise an das Tyrosin gebunden vorliegt. Erfindungsgemäß weiter bevorzugte Haarbehandlungsmittel sind dadurch gekennzeichnet, daß das in ihnen enthaltene Oligopeptid mindestens eine Aminosäuresequenz Leu-Tyr-Glu-Glu-Glu-Ile-Arg-Val-Leu aufweist, wobei die Amino-Gruppen frei oder protoniert und die Carboxy-Gruppen frei oder deprotoniert vorliegen können.
Ganz besonders bevorzugt enthalten erfindungsgemäße Mittel mindestens zwei Oligopeptide, die den vorstehend genannten Kriterien genügen, sich aber voneinander unterscheiden. So sind beispielsweise Haarbehandlungsmittel bevorzugt, die mindestens zwei voneinander verschiedene Oligopetide A und B enthalten, die beide die Aminosäuresequenz Tyr-Glu-Glu-Glu enthalten.

Ebenfalls noch weiter bevorzugte Haarbehandlungsmittel enthalten mindestens zwei voneinander verschiedene Oligopetide A und B, die beide die Aminosäuresequenz Tyr-Glu-Glu-Glu-Ile enthalten.

Ebenfalls ganz besonders bevorzugte Haarbehandlungsmittel enthalten mindestens zwei voneinander verschiedene Oligopetide A und B, die beide die Aminosäuresequenz Tyr-Glu-Glu-Glu-Ile-Arg enthalten.

Ebenfalls bevorzugte Ausführungsformen sind Haarbehandlungsmittel, die mindestens zwei voneinander verschiedene Oligopetide A und B enthalten, die beide die Aminosäuresequenz Tyr-Glu-Glu-Glu-Ile-Arg-Val enthalten.

Ebenfalls noch weiter bevorzugte Haarbehandlungsmittel enthalten mindestens zwei voneinander verschiedene Oligopetide A und B, die beide die Aminosäuresequenz Tyr-Glu-Glu-Glu-Ile-Arg-Val-Leu enthalten. Insbesondere bevorzugte Haarbehandlungsmittel sind dadurch gekennzeichnet, daß sie mindestens zwei voneinander verschiedene Oligopetide A und B enthalten, wobei das Oligopeptid A die Aminosäuresequenz Leu-Tyr-Glu-Glu-Glu-Ile-Arg-Val-Leu aufweist, wobei die Amino-Gruppen frei oder protoniert und die Carboxy-Gruppen frei oder deprotoniert vorliegen können und das Oligopeptid B die Aminosäuresequenz Tyr-Glu-Glu-Glu-Ile-Arg-Val-Leu aufweist, wobei die Amino-Gruppen frei oder protoniert und die Carboxy-Gruppen frei oder deprotoniert vorliegen können.
Ganz besonders bevorzugte Mittel dieser letztgenannten Ausführungsform enthalten - bezogen auf das Gewicht des Mittels - 0,00001 bis 1 Gew.% Oligopeptid A und 0,00001 bis 1 Gew.-% Oligopeptid B. Weiter bevorzugte Mittel dieser letztgenannten Ausführungsform enthalten - bezogen auf das Gewicht des Mittels -0,00005 bis 0,1 Gew.% Oligopeptid A und 0,00005 bis 0,1 Gew.% Oligopeptid B. Noch weiter bevorzugte Mittel dieser letztgenannten Ausführungsform enthalten - bezogen auf das Gewicht des Mittels -0,0001 bis 0,01 Gew.-% Oligopeptid A und 0,0001 bis 0,001 Gew.% Oligopeptid B.
Es hat sich gezeigt, daß der Einsatz der vorstehend genannten Oligopeptide den erfindungsgemäßen Haarbehandlungsmitteln herausragende Eigenschaften verleiht. Die erfindungsgemäßen Mittel verleihen den mit ihnen behandelten Haaren mehr Spannkraft, was sich in höheren Zugfestigkeiten der Keratinfasern und in einer Verringerung der Elastizitätsabnahme, z.B. bei Beschädigung durch atmosphärische Einwirkungen zeigt. Insbesondere die besonders bevorzugten Oligopeptide stabilisieren darüber hinaus den Feuchtigkeitshaushalt der keratinischen Fasern, so daß sich die Kämmbarkeit verbessert und der Alterungsprozess verzögert wird. Ein weiterer Vorteil der erfindungsgemäßen Zusammensetzungen liegt darin, daß die Formbarkeit und Restrukturierbarkeit von mit ihnen behandelten keratinischen Fasern verbessert wird. Die im Rahmen der vorliegenden Erfindung eingesetzten Oligopeptide, die den vorstehend genannten Bedingungen genügen, können vorteilhafterweise aus keratinischen Materialien gewonnen werden. Es ist erfindungsgemäß bevorzugt, daß diese Oligopeptide in hohen Anteilen, bezogen auf den gesamten keratinischen Peptidgehalt der Mittel, eingesetzt werden. Ganz besonders bevorzugt ist es, daß ein möglichst hoher Anteil aller im Mittel enthaltenen keratinischen Peptide den vorstehend genannten Bedingungen genügt.

Noch weiter bevorzugte erfindungsgemäße Haarbehandlungsmittel sind dadurch gekennzeichnet, daß mindestens 0,1 Gew.-%, vorzugsweise mindestens 0,5 Gew.-%, besonders bevorzugt mindestens 1 Gew.-%, weiter bevorzugt mindestens 2,5 Gew.-%, noch weiter bevorzugt mindestens 5 Gew.-% und insbesondere mindestens 10 Gew.-% aller im Mittel enthaltenen keratinischen Peptide die Aminosäuresequenz Tyr-Glu-Glu-Glu aufweisen. Besonders bevorzugte erfindungsgemäße Haarbehandlungsmittel sind dadurch gekennzeichnet, daß mindestens 0,1 Gew.-%, vorzugsweise mindestens 0,5 Gew.-%, besonders bevorzugt mindestens 1 Gew.-%, weiter bevorzugt mindestens 2,5 Gew.-%, noch weiter bevorzugt mindestens 5 Gew.-% und insbesondere mindestens 10 Gew.-% aller im Mittel enthaltenen keratinischen Peptide die Aminosäuresequenz Tyr-Glu-Glu-Glu-Ile aufweisen.
Ganz besonders bevorzugte erfindungsgemäße Haarbehandlungsmittel sind dadurch gekennzeichnet, daß mindestens 0,1 Gew.-%, vorzugsweise mindestens 0,5 Gew.-%, besonders bevorzugt mindestens 1 Gew.-%, weiter bevorzugt mindestens 2,5 Gew.-%, noch weiter bevorzugt mindestens 5 Gew.-% und insbesondere mindestens 10 Gew.-% aller im Mittel enthaltenen keratinischen Peptide die Aminosäuresequenz Tyr-Glu-Glu-Glu-Ile-Arg aufweisen. Noch weiter bevorzugte erfindungsgemäße Haarbehandlungsmittel sind dadurch gekennzeichnet, daß mindestens 0,1 Gew.-%, vorzugsweise mindestens 0,5 Gew.-%, besonders bevorzugt mindestens 1 Gew.-%, weiter bevorzugt mindestens 2,5 Gew.-%, noch weiter bevorzugt mindestens 5 Gew.-% und insbesondere mindestens 10 Gew.-% aller im Mittel enthaltenen keratinischen Peptide die Aminosäuresequenz Tyr-Glu-Glu-Glu-Ile-Arg-Val aufweisen. Insbesondere bevorzugte erfindungsgemäße Haarbehandlungsmittel sind dadurch gekennzeichnet, daß mindestens 0,1 Gew.-%, vorzugsweise mindestens 0,5 Gew.-%, besonders bevorzugt mindestens 1 Gew.-%, weiter bevorzugt mindestens 2,5 Gew.-%, noch weiter bevorzugt mindestens 5 Gew.-% und insbesondere mindestens 10 Gew.-% aller im Mittel enthaltenen keratinischen Peptide die Aminosäuresequenz Tyr-Glu-Glu-Glu-Ile-Arg-Val-Leu aufweisen.
Die vorstehend genannten Bedingungen betreffen den Gesamtgehalt der erfindungsgemäßen Mittel an Peptiden, welche aus keratinischen Materalien stammen. Zusätzlich zu den Oligopeptiden keratinischer Herkunft können selbstverständlich weitere Peptide und/oder Protenihydrolysate eingesetzt werden, beispielsweise aus anderen nativen Quellen. Bevorzugt ist beispielsweise der zusätzliche Einsatz von Weizenproteinydrolysaten, siehe weiter unten.

Die erfindungsgemäßen Mittel können weitere Wirk- und Hilfsstoffe beinhalten. Diese werden nachfolgend beschrieben.
Vorzugsweise enthalten die erfindungsgemäßen Mittel zusätzlich mindestens einen Emulgator bzw. ein Tensid, wobei oberflächenaktive Substanzen je nach Anwendungsgebiet als Tenside oder als Emulgatoren bezeichnet werden und aus anionischen, kationischen, zwitterionischen, ampholytischen und nichtionischen Tensiden und Emulgatoren ausgewählt sind. Erfindungsgemäß bevorzugte Haarbehandlungsmittel sind dadurch gekennzeichnet, daß sie - bezogen auf sein Gewicht - 0,5 bis 70 Gew.-%, vorzugsweise 1 bis 60 Gew.-% und insbesondere 5 bis 25 Gew.-% anionische(s) und/oder nichtionische(s) und/oder kationische(s) und/oder amphotere(s) Tensid(e), enthelten.
Ganz besonders bevorzugt sind erfindungsgemäße Mittel, die zusätzlich Fettalkohol(e) und/oder Fettalkoholalkoxylat(e), vorzugsweise C₁₂₋₂₂-Fettalkohol(e) und/oder C₁₂₋₂₂-Fettalkoholethoxylat(e) mit 10 bis 30 EO-Einheiten, besonders bevorzugt C₁₆₋₁₈-Fettalkohol(e) und/oder C₁₆₋₁₈-Fettalkoholethoxylat(e) mit 12 bis 20 EO-Einheiten, vorzugsweise in Mengen von 5 bis 20 Gew.-%, bevorzugt von 7,5 bis 17,5 Gew.-% und insbesondere von 10 bis 15 Gew.-%, jeweils bezogen auf das Gewicht des Mittels, enthalten. Zusammenfassend sind erfindungsgemäße Haarbehandlungsmittel bevorzugt, die - bezogen auf ihr Gewicht - 0,1 bis 20 Gew.-%, vorzugsweise 0,25 bis 17,5 Gew.-% und insbesondere 5 bis 15 Gew.-% anionische(s) Tensid(e), besonders bevorzugt Fettalkoholethersulfate der Formel

**H₃C-(CH₂)ₙ-(OCH₂CH₂)ₖ-OSO₃⁻ M⁺**

enthalten, in der n für Werte von 5 bis 21, vorzugsweise von 7 bis 19, besonders bevorzugt von 9 bis 17 und insbesondere von 11 bis 13 und k für Werte von 1, 2, 3, 4, 5, 6, 7, 8, 9 oder 10, vorzugsweise für 1, 2 oder 3 und insbesondere für 2 stehen, und M für ein Kation aus der Gruppe Na⁺, K⁺ NH₄⁺, ½ Mg²⁺, ½ Zn²⁺, vorzugsweise für Na⁺, stehen.
Bevorzugte erfindungsgemäße Haarbehandlungsmittel sind weiter dadurch gekennzeichnet, daß sie zusätzlich amphotere(s) Tensid(e), vorzugsweise aus den Gruppen der N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren mit jeweils etwa 8 bis 24 C-Atomen in der Alkylgruppe,Alkylaminoessigsäuren mit jeweils etwa 8 bis 24 C-Atomen in der Alkylgruppe, N-Kokosalkylaminopropionat, Kokosacylaminoethylaminopropionat, C₁₂-C₁₈ - Acylsarcosin, N-Alkyl-N,N-dimethylammonium-glycinate, beispielsweise Kokosalkyl-dimethylammoniumglycinat, N-Acyl-aminopropyl-N,N-dimethylammoniumglycinate, beispielsweise Kokosacylaminopropyl-dimethylammoniumglycinat, 2-Alkyl-3-carboxymethyl-3-hydroxyethyl-imidazoline mit jeweils 8 bis 18 C-Atomen in der Alkyl- oder Acylgruppe, Kokosacylaminoethylhydroxyethylcarboxymethylglycinat, der unter der INCI-Bezeichnung Cocamidopropyl Betain bekannten Verbindungen, der unter der INCI-Bezeichnung Disodium Cocoamphodiacetate bekannten Verbindungen, enthalten, wobei bevorzugte Mittel das bzw. die amphotere(n) Tensid(e) in Mengen von 1 bis 15 Gew.-%, vorzugsweise von 2,5 bis 12 Gew.-% und insbesondere von 5 bis 10 Gew.-%, jeweils bezogen auf das gesamte Mittel, enthalten.
Als weiteren optionalen Bestandteil können die erfindungsgemäßen Mittel 0,01 bis 10 Gew.-% mindestens eines Polymers aus der Gruppe der kationischen und/oder amphoteren Polymere enthalten. Vorzugsweise wird das Polymer bzw. werden die Polymere innerhalb engerer Mengenbereiche eingesetzt. So sind erfindungsgemäße Mittel bevorzugt, die - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% amphotere(s) Polymer(e), enthalten.
Unabhängig davon, ob in den Mitteln amphotere Polymere enthalten sind oder nicht, sind weiter bevorzugte erfindungsgemäße Mittel dadurch gekennzeichnet, daß sie - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthalten.
Erfindungsgemäß bevorzugt einsetzbare kationische Polymere sind im Prioritätsdokument auf den Seiten 21 bis 25 beschrieben:
Zusammenfassend sind erfindungsgemäße Haarbehandlungsmittel bevorzugt, die - bezogen auf ihr Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthalten, wobei bevorzugte kationische(s) Polymer(e) ausgewählt ist/sind aus Poly(methacryloyloxyethyltrimethylammoniumchlorid) (INCI: Polyquaternium-37) und/oder quaternisierten Cellulose-Derivaten (INCI: Polyquaternium 10) und/oder kationischen Alkylpolyglycosiden und/oder kationisertem Honig und/oder kationischen Guar-Derivaten und/oder polymeren Dimethyldiallylammoniumsalzen und deren Copolymeren mit Estern und Amiden von Acrylsäure und Methacrylsäure und/oder Copolymeren des Vinylpyrrolidons mit quaternierten Derivaten des Dialkylaminoalkylacrylats und -methacrylats und/oder Vinylpyrrolidon-Vinylimidazoliummethochlorid-Copolymeren und/oder quaterniertem Polyvinylalkohol und/oder Polyquaternium 2 und/oder Polyquaternium-7 und/oder Polyquaternium 17 und/oder Polyquaternium 18 und/oder Polyquaternium 24 und/oder Polyquaternium 27.

Zusätzlich zu den kationischen Polymeren oder an ihrer Stelle können die erfindungsgemäßen Mittel amphotere Polymere enthalten. Im Rahmen der vorliegenden Erfindung bevorzugt einsetzbare amphotere Polymerisate sind im Prioritätsdokument auf den Seiten 25 bis 30 beschrieben.
Eine weitere bevorzugte Gruppe von Inhaltsstoffen der erfindungsgemäßen Mittel sind Vitamine, Provitamine oder Vitaminvorstufen. Zusammenfassend sind erfindungsgemäße Haarbehandlungsmittel bevorzugt, die zusätzlich als Pflegestoff - bezogen auf sein Gewicht - 0,1 bis 5 Gew.-%, vorzugsweise 0,2 bis 4 Gew.-%, besonders bevorzugt 0,25 bis 3,5 Gew.-%, weiter bevorzugt 0,5 bis 3 Gew.-% und insbesondere 0,5 bis 2,5 Gew.-% Vitamine und/oder Pro-Vitamine und/oder Vitaminvorstufen enthalten, die vorzugsweise den Gruppen A, B, C, E, F und H zugeordnet werden, wobei bevorzugte Mittel Panthenol ((±)-2,4-Dihydroxy-*N*-(3-hydroxypropyl)-3,3-dimethyl-butyramid, Provitamin B₅) und/oder Pantothensäure (Vitamin B₃, Vitamin B₅) und/oder Niacin, Niacinamid bzw. Nicotinamid (Vitamin B₃) und/oder L-Ascorbinsäure (Vitamin C) und/oder Thiamin (Vitamin B₁) und/oder Riboflavin (Vitamin B₂, Vitamin G) und/oder Biotin (Vitamin B₇, Vitamin H) und/oder Folsäure (Vitamin B₉, Vitamin B_{c} oder Vitamin M) und/oder Vitamin B₆ und/oder Vitamin B₁₂ enthalten.
Es hat sich gezeigt, daß der Einsatz bestimmter Chinone eine Antischuppen- und Anti-Haarausfallwirkung verstärkt sowie Vorteile in Bezug auf Kämmbarkeit und Glanz bewirkt. Als weiteren Bestandteil können die erfindungsgemäßen Mittel daher 0,0001 bis 5 Gew.-% mindestens eines Biochinons der Formel (I) 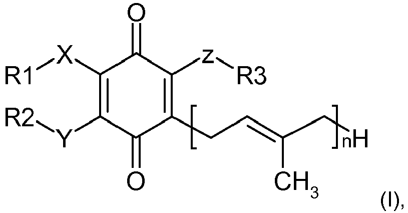 enthalten in der
- X, Y, Z: stehen unabhängig voneinander für -O- oder -NH- oder NR⁴- oder eine chemische Bindung
- R¹, R², R³: stehen unabhängig voneinander für ein Wasserstoffatom oder eine gegebenenfalls substituierte Arylgruppe oder eine gegebenenfalls substituierte (C₁-C₆)-Alkylgruppe oder eine Hydroxyalkylgruppe oder eine Polyhydroxyalkylgruppe oder eine gegebenenfalls substituierte (C₁-C₆)-Alkylengruppe, oder einen (C₁-C₆)-Acylrest, wobei bevorzugte Reste unabhängig voneinander ausgewählt sind aus -H, -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- R⁴: steht für -CH₃, -CH₂CH₃, -(CH₂)₂CH₂, -CH(CH₃)₂, -(CH₂)₃CH₃, -CH(CH₃)CH₂CH₃, -CH₂CH(CH₃)₂, -C(CH₃)₃
- n: steht für Werte von 1 bis 20, vorzugsweise von 2 bis 15 und insbesondere für 5, 6, 7, 8, 9, 10.

Besonders bevorzugte erfindungsgemäße Haarbehandlungsmittel sind dadurch gekennzeichnet, daß sie als Pflegestoff - bezogen auf ihr Gewicht - 0,0001 bis 1 Gew.-%, bevorzugt 0,001 bis 0,5 Gew.-% und besonders bevorzugt 0,005 bis 0,1 Gew.-% mindestens eines Ubichinons und/oder mindestens eines Ubichinols und/oder mindestens eines Derivates dieser Substanzen enthalten, wobei bevorzugte Mittel ein Ubichinon der Formel (Ubi) enthalten 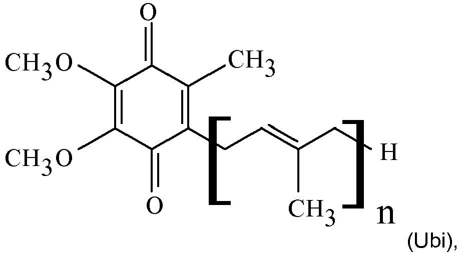 in der n für die Werte = 6, 7, 8, 9 oder 10, besonders bevorzugt für 10 (Coenzym Q10) steht. Alternativ zu den besonders bevorzugten Ubichinonen oder zusätzlich zu ihnen können die erfindungsgemäßen Mittel auch Plastochinone enthalten. Hier sind bevorzugte erfindungsgemäße Mittel dadurch gekennzeichnet, daß sie 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% mindestens eines Plastochinons der Formel (Ib) enthalten 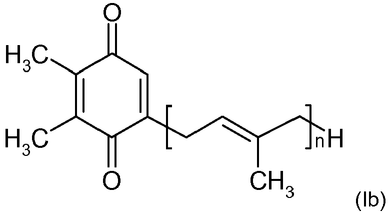 in der n für Werte von 1 bis 20, vorzugsweise von 2 bis 15 und insbesondere für 5, 6, 7, 8, 9, 10 steht, wobei besonders bevorzugt Mittel Plastochinon PQ-9 (n = 9) enthalten.
Zur Verbesserung der Elastizität und Festigung der inneren Struktur der mit erfindungsgemäßen Mitteln behandelter Haare können die erfindungsgemäßen Mittel Purin und/oder Purinderivate enthalten. Insbesondere die Kombination von Purin und/oder Purinderivaten mit Ubichinonen und/oder Plastochinonen führt dazu, daß die mit entsprechenden Mitteln behandelten Haare unter anderem höhere Meßwerte bei der Differenzthermoanalyse und verbesserte Naß- und Trockenkämmbarkeiten zeigen.
Als weiteren Inhaltsstoff können die erfindungsgemäßen Mittel daher Purin und/oder Derivat(e) des Purins enthalten. Bevorzugte erfindungsgemäße Mitteln enthalten Purin und/oder Purinderivate in engeren Mengenbereichen. Hier sind erfindungsgemäß bevorzugte kosmetische Mittel dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthalten.
Unter Purin, den Purinen und den Purinderivaten sind erfindungsgemäß einige Vertreter besonders bevorzugt. Erfindungsgemäß bevorzugte Haarbehandlungsmittel sind dadurch gekennzeichnet, daß sie als Pflegesgtoff - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Purin(e) und/oder Purinderivat(e) enthält, wobei bevorzugte Mittel Purin und/oder Purinderivat(e) der Formel (Pur-I) enthalten 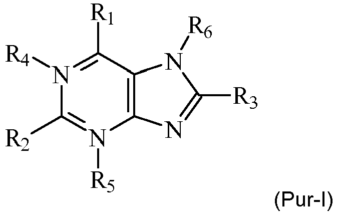 in der die Reste R¹, R² und R³ unabhängig voneinander ausgewählt sind aus -H, - OH, NH₂, -SH und die Reste R⁴, R⁵ und R⁶ unabhängig voneinander ausgewählt sind aus -H, -CH₃ und -CH₂-CH₃, wobei folgende Verbindungen bevorzugt sind: Purin (R¹ = R² = R³ = R⁴ = R⁵ = R⁶ = H), Adenin (R¹ = NH₂, R² = R³ = R⁴ = R⁵ = R⁶ = H), Guanin (R¹ = OH, R² = NH₂, R³ = R⁴ = R⁵ = R⁶ = H), Harnsäure (R¹ = R² = R³ = OH, R⁴ = R⁵ = R⁶ = H), Hypoxanthin (R¹ = OH, R² = R³ = R⁴ = R⁵ = R⁶ = H), 6-Purinthiol (R¹ = SH, R² = R³ = R⁴ = R⁵ = R⁶ = H), 6-Thioguanin (R¹ = SH, R² = NH₂, R³ = R⁴ = R⁵ = R⁶ = H), Xanthin (R¹ = R² = OH, R³ = R⁴ = R⁵ = R⁶ = H), Coffein (R¹ = R² = OH, R³ = H, R⁴ = R⁵ = R⁶ = CH₃), Theobromin (R¹ = R² = OH, R³ = R⁴ = H, R⁵ = R⁶ = CH₃), Theophyllin (R¹ = R² = OH, R³ = H, R⁴ = CH₃, R⁵ = CH₃, R⁶ = H).
Je nach gewünschtem Anwendungszweck der erfindungsgemäßen Mittel kann dabei die Art und Menge des Purinderivates variieren. In haarkosmetischen Formulierungen hat sich insbesondere Coffein bewährt, das beispielsweise in Shampoos vorzugsweise in Mengen von 0,005 bis 0,25 Gew.-%, weiter bevorzugt von 0,01 bis 0,1 Gew.-% und insbesondere von 0,01 bis 0,05 Gew.-% (jeweils bezogen auf das Shampoo) eingesetzt werden kann.

Es ist weiterhin vorteilhaft, Purin bzw. Purinderivate und Biochinone in einem bestimmten Verhältnis zueinander einzusetzen. Hier sind erfindungsgemäße Mittel bevorzugt, bei denen das Gewichtsverhältnis der Inhaltsstoffe a) und b) 10:1 bis 1:100, vorzugsweise 5:1 bis 1:50, besonders bevorzugt 2:1 bis 1:20 und insbesondere 1:1 bis 1:10 beträgt.
Wie bereits erwähnt, ist Coffein ein besonders bevorzugtes Purinderivat, und das Coenzym Q10 ist ein besonders bevorzugtes Biochinon. Besonders bevorzugte erfindungsgemäße Mittel sind daher dadurch gekennzeichnet, daß sie - bezogen auf ihr Gewicht - 0,001 bis 2,5 Gew.-%, vorzugsweise 0,0025 bis 1 Gew.-%, besonders bevorzugt 0,005 bis 0,5 Gew.-% und insbesondere 0,01 bis 0,1 Gew.-% Coffein und 0,0002 bis 4 Gew.-%, vorzugsweise 0,0005 bis 3 Gew.-%, besonders bevorzugt 0,001 bis 2 Gew.-%, weiter bevorzugt 0,0015 bis 1 und insbesondere 0,002 bis 0,5 Gew.-% Coenzym Q10 enthalten.
Als weiteren Bestandteil können die erfindungsgemäßen Mittel mindestens ein Kohlenhydrat aus der Gruppe der Monosaccharide, Disaccharide und/oder Oligosaccharide enthalten. Hier sind erfindungsgemäß bevorzugte Haarbehandlungsmittel dadurch gekennzeichnet, daß sie als Pflegesgtoff - bezogen auf ihr Gewicht - 0,01 bis 5 Gew.-%, vorzugsweise 0,05 bis 4,5 Gew.-%, besonders bevorzugt 0,1 bis 4 Gew.-%, weiter bevorzugt 0,5 bis 3,5 Gew.-% und insbesondere 0,75 bis 2,5 Gew.-% Kohlenhydrat(e), ausgewählt aus Monosacchariden, Disacchariden und/oder Oligosacchariden enthalten, wobei bevorzugte Kohlenhydrate ausgewählt sind aus onosachhariden (insbesondere D-Ribose und/oder D-Xylose und/oder L-Arabinose und/oder D-Glucose und/oder D-Mannose und/oder D-Galactose und/oder D-Fructose und/oder Sorbose und/oderL-Fucose und/oder L-Rhamnose) und/oder Disacchariden (insbesondere Saccharose und/oder Maltose und/ode rLactose und/oder Trehalose und/oder Cellobiose und/oder Gentiobiose und/oder Isomaltose).
Besonders bevorzugte erfindungsgemäße Mittel enthalten bezogen auf ihr Gewicht
- 0,005 bis 0,015 Gew.-% Coffein und 0,75 bis 1,5 Gew.-% Glucosemonohydrat,
- 0,005 bis 0,015 Gew.-% Coffein und 0,75 bis 1,5 Gew.-% Saccharose,
- 0,005 bis 0,015 Gew.-% Coffein und 0,75 bis 1,5 Gew.-% Fructose.
Wie bereits erwähnt, enthalten bevorzugte erfindungsgemäße Mittel (eine) Aminosäure(n). Erfindungsgemäß besonders bevorzugt einsetzbare Aminosäuren stammen aus der Gruppe Glycin, Alanin, Valin, Leucin, Isoleucin, Phenylalanin, Tyrosin, Tryptophan, Prolin, Asparaginsäure, Glutaminsäure, Asparagin, Glutamin, Serin, Threonin, Cystein, Methionin, Lysin, Arginin, Histidin, β-Alanin, 4-Aminobuttersäure (GABA), Betain, L-Cystin (L-Cyss), L-Carnitin, L-Citrullin, L-Theanin, 3 ',4 '-Dihydroxy-L-phenylalanin (L-Dopa), 5 '-Hydroxy-L-tryptophan, L-Homocystein, S-Methyl-L-methionin, S-Allyl-L-cystein-sulfoxid (L-Alliin), L-*trans*-4-Hydroxyprolin, L-5-Oxoprolin (L-Pyroglutaminsäure), L-Phosphoserin, Kreatin, 3-Methyl-L-histidin, L-Ornithin, wobei sowohl die einzelnen Aminosäuren als auch Mischungen eingesetzt werden können.

Bevorzugte erfindungsgemäße Mittel enthalten eine oder mehrere Aminosäuren in engeren Mengenbereichen. Hier sind erfindungsgemäß bevorzugte kosmetische Mittel dadurch gekennzeichnet, daß sie zusätzlich - 0,05 bis 5 Gew.-%, vorzugsweise 0,1 bis 2,5 Gew.-%, besonders bevorzugt 0,15 bis 1 Gew.-% und insbesondere 0,2 bis 0,5 Gew.-% Aminosäure(n), vorzugsweise (eine) Aminosäure(n) aus der Gruppe Glycin und/oder Alanain und/oder Valin und/oder Lysin und/oder Leucin und/oder Threonin enthalten.
Erfindungsgemäß bevorzugte Mittel enthalten als Pflegestoff - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% mindestens eines 2-Furanonderivats der Formel (Fur-I) und/oder der Formel (Fur-II) 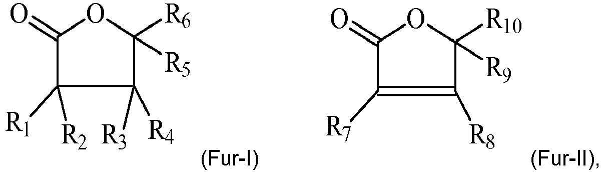 in welchen die Reste R¹ bis R¹⁰ unabhängig voneinander stehen für:
- Wasserstoff, -OH, einen Methyl-, Methoxy-, Aminomethyl- oder Hydroxymethylrest,
- -C₂ - C₄ - gesättigten oder ein- oder zweifach ungesättigten, verzweigten oder linearen Kohlenwasserstoffrest,
- -C₂ - C₄ - gesättigten oder ein- oder zweifach ungesättigten, verzweigten oder linearen Mono-, Di- oder Trihydroxykohlenwasserstoffrest,
- -C₂ - C₄ - gesättigten oder ein- oder zweifach ungesättigten, verzweigten oder linearen Mono-, Di- oder Triaminokohlenwasserstoffrest,
- eine Gruppe -OR¹¹, mit R¹¹ als einem -C₂ - C₄ - gesättigten oder ein- oder zweifach ungesättigten, verzweigten oder linearen Kohlenwasserstoffrest, -C₂ - C₄ - gesättigten oder ein- oder zweifach ungesättigten, verzweigten oder linearen Mono-, Di- oder Trihydroxykohlenwasserstoffrest,
- eine Gruppe -NR¹²R¹³, wobei R¹² und R¹³ jeweils unabhängig voneinander stehen für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest,
- eine Gruppe -COOR¹⁴, wobei R¹⁴ steht für Wasserstoff, einen Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -CONR¹⁵R¹⁶, wobei R¹⁵ und R¹⁶ jeweils stehen für Wasserstoff, Methyl-, einen -C₂ - C₄ - gesättigten oder ein- oder zweifach ungesättigten, verzweigten oder linearen Kohlenwasserstoffrest, einen -C₂ - C₄ - gesättigten ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -COR¹⁶, wobei R¹⁶ steht für einen Methyl-, einen -C₂ - C₄ - gesättigten oder ein-oder zweifach ungesättigten, verzweigten oder linearen Kohlenwasserstoffrest, einen -C₂ - C₄ - gesättigten oder ein- oder zweifach ungesättigten, verzweigten oder linearen Mono-, Di- oder Trihydroxykohlenwasserstoffrest, einen -C₂ - C₄ - gesättigten oder ein- oder zweifach ungesättigten, verzweigten oder linearen Mono-, Di- oder Triaminokohlenwasserstoffrest,
- eine Gruppe -OCOR¹⁷, wobei R¹⁷ steht für einen Methyl-, einen -C₂ - C₃₀ - gesättigten oder ein- oder mehrfach ungesättigten, verzweigten oder linearen Kohlenwasserstoffrest, einen -C₂ - C₃₀ - gesättigten oder ein- oder mehrfach ungesättigten, verzweigten oder linearen Mono-, Di-, Tri- oder Polyhydroxykohlenwasserstoffrest, einen -C₂ - C₃₀ - gesättigten oder ein- oder mehrfach ungesättigten, verzweigten oder linearen Mono-, Di-, Tri- oder Polyaminokohlenwasserstoffrest,
mit der Maßgabe, daß für den Fall, wenn R⁷ und R⁸ für -OH und gleichzeitig R⁹ oder R¹⁰ für Wasserstoff stehen, die verbleibende Gruppe R⁹ oder R¹⁰ nicht für einen Dihydroxyethylrest steht. In einer ganz besonders bevorzugten Ausführungsform der erfindungsgemäßen Lehre wird als Verbindung entsprechend der Formel (Fur-I) Dihydro-3-hydroxy-4,4-dimethyl-2(3H)-furanon eingesetzt.

Ein weiterer, bevorzugter einsetzbarer Pflegestoff, der aktivierende Eigenschaften besitzt, ist das taurin. Erfindungsgemäß bevorzugte Haarbehandlungsmittel enthalten als Pflegestoff - bezogen auf ihr Gewicht - 0,01 bis 15 Gew.-%, vorzugsweise 0,025 bis 12,5 Gew.-%, besonders bevorzugt 0,05 bis 10 Gew.-%, weiter bevorzugt 0,1 bis 7,5 Gew.-% und insbesondere 0,5 bis 5 Gew.-% Taurin (2-Aminoethansulfonsäure).
Bevorzugt ist auch der zusätzlich Einsatz von Bisabolol und/oder Bisabololoxiden in den erfindungsgemäßen Mitteln. Hier sind erfindungsgemäße Haarbehandlungsmittel bevorzugt, die zusätzlich 0,001 bis 5 Gew.-%, vorzugsweise 0,01 bis 4 Gew.-%, besonders bevorzugt 0,02 bis 2,5 Gew.-% und insbesondere 0,1 bis 1,5 Gew.-% Bisabolol und/oder Oxide von Bisabolol, vorzugsweise (-)-alpha-Bisabolol enthalten.
Die erfindungsgemäßen Mittel können zusätzlich zu dem/den Oligopeptid(en) und optionalen weiteren Inhaltsstoffen weitere Stoffe enthalten, die Haarausfall verhindern, lindern oder heilen. Insbesondere ist ein Gehalt an haarwurzelstabilisierenden Wirkstoffen vorteilhaft.

Zusammenfassend sind erfindungsgemäße kosmetische Mittel bevorzugt, die zusätzlich - bezogen auf sein Gewicht - 0,001 bis 5 Gew.-% Haarwurzel-stabilisierende Stoffe, insbesondere Minoxidil und/oder Finasterid und/oder Ketoconazol enthalten.
Durch zusätzliche Antischuppenwirkstoffe (beispielsweise Climbazol, Piroctone Olamine oder Zink-Pyrithion) wird die Menge des Schuppen verursachenden Hefepilzes gezielt reduziert, die Keimflora erreicht wieder die normale prozentuale Zusammensetzung und die Abschuppung wird auf das physiologische Maß reduziert. Labortests haben jedoch nachgewiesen, daß die unterschiedlichen Artvertreter des Pityrosporum ovale unterschiedlich gut auf die Antischuppenwirkstoffe reagieren. Um alle Schuppenerreger maximal zu bekämpfen ist daher eine Kombination von Anti-Schuppenwirkstoffen am erfolgreichsten.
Zusammenfassend sind erfindungsgemäße Haarbehandlungsmittel bevorzugt, die zusätzlich - bezogen auf ihr Gewicht - 0,001 bis 5 Gew.-% Antischuppenwirkstoffe, insbesondere Piroctone Olamine (1-Hydroxy-4-methyl-6-(2,4,4-trirnethylpentyl)pyridin-2(1H)-on, Verbindung mit 2-Aminoethanol, 1:1) und/oder Zink-Pyrithion und/oder Selensulfid und/oder Climbazol und/oder Salicylsäure oder Fumarsäure enthalten.
Die erfindungsgemäßen Mittel können weiterhin alle für solche Zubereitungen bekannten Wirk-, Zusatz- und Hilfsstoffe enthalten.
Als weiterhin vorteilhaft hat es sich gezeigt, wenn zusätzlich zu dem bzw. den Polymer(en) aus der Gruppe der kationischen und/oder amphoteren Polymere weitere Polymere (G) in den erfindungsgemäßen Mitteln enthalten sind. In einer bevorzugten Ausführungsform werden den erfindungsgemäßen Mitteln daher weitere Polymere zugesetzt, wobei sich sowohl anionische als auch nichtionische Polymere als wirksam erwiesen haben.
Besonders bevorzugt ist das Homopolymer der 2-Acrylamido-2-methylpropansulfonsäure, das beispielsweise unter der Bezeichnung Rheothik^{®}11-80 im Handel erhältlich ist.
Ein weiteres Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen. Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.
Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.
Die erfindungsgemäßen Mittel können in einer weiteren Ausführungsform nichtionogene Polymere (G4) enthalten. Geeignete nichtionogene Polymere sind beispielsweise:
- Vinylpyrrolidon/Vinylester-Copolymere,
- Celluloseether, wie Hydroxypropylcellulose, Hydroxyethylcellulose
- Stärke und deren Derivate, insbesondere Stärkeether,
- Schellack
- Polyvinylpyrrolidone,
- Siloxane
- Glycosidisch substituierte Silicone.
Es ist erfindungsgemäß auch möglich, daß die Zubereitungen mehrere, insbesondere zwei verschiedene Polymere gleicher Ladung und/oder jeweils ein ionisches und ein amphoteres und/oder nicht ionisches Polymer enthalten. Die weiteren Polymere (G) sind in den erfindungsgemäßen Mitteln bevorzugt in Mengen von 0,05 bis 10 Gew.-%, bezogen auf das gesamte Mittel, enthalten. Mengen von 0,1 bis 5, insbesondere von 0,1 bis 3 Gew.-%, sind besonders bevorzugt.
Eine besonders bevorzugte Gruppe von Inhaltsstoffen stellen die Silikone dar. Erfindungsgemäße bevorzugte Mittel sind dadurch gekennzeichnet, daß sie mindestens ein Silicon, vorzugsweise ein Silicon enthalten, das ausgewählt ist unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopoylmeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan-Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
oder deren Gemischen.

Erfindungsgemäß besonders bevorzugte Mittel enthalten das bzw. die Silikon(e) vorzugsweise in Mengen von 0,1 bis 10 Gew.-%, vorzugsweise von 0,25 bis 7 Gew.-% und insbesondere von 0,5 bis 5 Gew.-%, jeweils bezogen auf das gesamte Mittel.
Besonders bevorzugte erfindungsgemäße Mittel sind dadurch gekennzeichnet, daß sie mindestens ein Silikon der Formel Si-I

(CH₃)₃Si-[O-Si(CH₃)₂]ₓ-O-Si(CH₃)₃ (Si-I),

enthalten, in der x für eine Zahl von 0 bis 100, vorzugsweise von 0 bis 50, weiter bevorzugt von 0 bis 20 und insbesondere 0 bis 10, steht. Diese Silikone werden nach der INCI-Nomenklatur als DIMETHICONE bezeichnet.
Bevorzugte erfindungsgemäß einsetzbare Silikone weisen bei 20°C Viskositäten von 0,2 bis 2 mm²s⁻¹ auf, wobei Silikone mit Viskositäten von 0,5 bis 1 mm²s⁻¹ besonders bevorzugt sind. Besonders bevorzugte erfindungsgemäße Mittel enthalten ein oder mehrere aminofunktionelle Silicone. Unabhängig davon, welche aminofunktionellen Silicone eingesetzt werden, sind erfindungsgemäße Mittel bevorzugt, die ein aminofunktionalles Silikon enthalten dessen Aminzahl oberhalb von 0,25 meq/g, vorzugsweise oberhalb von 0,3 meq/g und insbesondere oberhalb von 0,4 meq/g liegt. Erfindungsgemäß bevorzugte Mittel sind dadurch gekennzeichnet, daß sie, bezogen auf ihr Gewicht, 0,01 bis 10 Gew.%, vorzugsweise 0,1 bis 8 Gew.%, besonders bevorzugt 0,25 bis 7,5 Gew.% und insbesondere 0,5 bis 5 Gew.% aminofunktionelle(s) Silikon(e) enthalten. Auch die nach INCI als CYCLOMETHICONE bezeichneten cyclischen Dimethicone sind erfindungsgemäß mit Vorzug einsetzbar. Hier sind erfindungsgemäße Mittel bevorzugt, die mindestens ein Silikon der Formel Si-III 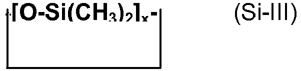 enthalten, in der x für eine Zahl von 3 bis 200, vorzugsweise von 3 bis 10, weiter bevorzugt von30 bis 7 und insbesondere 3, 4, 5 oder 6, steht.

Aus ästhetischen Gründen werden "klare" Produkte von Verbrauchern oft bevorzugt. Erfindungsgemäß bevorzugte Haarbehandlungsmittel sind daher dadurch gekennzeichnet, daß sie transparent bzw. transluzent sind. Unter transparent oder transluzent wird im Rahmen der vorliegenden Erfindung eine Zusammensetzung verstanden, die einen NTU-Wert von unter 100 aufweist. Der NTU-Wert (Nephelometric Turbidity Unit, *Nephelometrischer Trübungswert*; NTU) ist eine in der Wasseraufbereitung verwendete Einheit für Trübungsmessungen in Flüssigkeiten. Sie ist die Einheit einer mit einem kalibrierten Nephelometer gemessenen Trübung einer Flüssigkeit. Die erfindungsgemäßen Mittel weisen vorteilhafte Eigenschaften auf und verleihen den mit ihnen behandelten Haaren ebenfalls vorteilhafte Eigenschaften. Insbesondere bei der Haar- und Kopfhautbehandlung wurden Vorteile beobachtet. So steigern erfindungsgemäße Haarbehandlungsmittel die Elastizität der mit ihnen behandelten Haare und führen zu einer innerstrukturellen Stärkung der Haarfasern, welche sich z.B. in höheren Schmelztemperaturen bei der Differenzthermoanalyse niederschlägt.
Es zeigt sich auch eine Verbesserung der Naß- und Trockenkämmbarkeiten sowie eine Verhinderung frühzeitiger Splißbildung bei den behandelten Haaren. Auf der Haut und insbesondere der Kopfhaut bewirken die erfindungsgemäßen Mittel eine Erhöhung der Elastizität und überraschenderweise sebumregulierende Effekte. Der optische Eindruck "fettiger" Haut oder Haare wird damit vermieden bzw. abgeschwächt.
Ein weiterer Gegenstand der vorliegenden Erfindung die Verwendung von erfindungsgemäßen Mitteln zur Verbesserung mindestens einer der Eigenschaften
- Zugfestigkeit von keratinischen Fasern, insbesondere menschlichen Haaren;
- Stabilisierung des Feuchtigkeitshaushaltes von keratinischen Fasern, insbesondere menschlichen Haaren;
- Kämmbarkeit von keratinischen Fasern, insbesondere menschlichen Haaren;
- Verzögerung des Alterungsprozesses von keratinischen Fasern, insbesondere menschlichen Haaren;
- Restrukturierbarkeit von keratinischen Fasern, insbesondere menschlichen Haaren, während des und nach dem Dauerwellprozeß;
- Verringerung der Elastizitätsabnahme von keratinischen Fasern, insbesondere menschlichen Haaren bei Beschädigung durch atmosphärische Einwirkungen.
Bezüglich weiterer bevorzugter Ausführungsformen der erfindungsgemäßen Verwendung gilt mutatis mutandis das zu den erfindungsgemäßen Mitteln Gesagte.
Die nachstehenden Beispiele sollen den Gegenstand der Erfindung näher erläutern, ohne ihn zu beschränken.

### Beispiele:

| **Shampoo** | Gew.-% |
|---|---|
| Citronensäure wasserfrei | 0,5 |
| Natriumlaurylethersulfat.25% | 50 |
| Disodium Cocoamphodiacetate | 7 |
| Salicylsäure | 0,2 |
| D-Panthenol 75 % | 0,5 |
| Na-benzoat | 0,5 |
| Euperlan PK 3000 AM | 2 |
| Cetiol HE | 1 |
| **Oligopeptid-Mischung^{#}** | **0,01** |
| Polymer JR 400 | 0,5 |
| Parfüm | 0,5 |
| PEG-40 Hydrogenated Castor Oil 455 | 1 |
| Macadamianussöl, raffiniert | 0,2 |
| Natriumchlorid fein-mittel | 0,5 |
| Wasser, vollentsalzt | ad 100 |

- #: enthaltend - bezogen auf ihr Gewicht - mindestens 10 Gew.-% einer Mischung aus Oligopeptiden aus 8 Aminosäuren mit der Aminosäuresequenz Tyr-Glu-Glu-Glu-Ile-Arg-Val-Leu und Oligopeptiden aus 9 Aminosäuren mit der Aminosäuresequenz Leu-Tyr-Glu-Glu-Glu-Ile-Arg-Val-Leu
- Euperlan^{®} PK3000: ca. 60-64% Festkörper; INCI-Bezeichnung: Glycol Distearate, Glycerin, Laureth-4, Cocamidopropyl Betaine (Cognis)
- Cetiol^{®} HE: Kokosmonglycerid mit ca. 7,3 EO-Einheiten (INCI-Bezeichnung: PEG-7 Glyceryl Cocoate) (Cognis)
- Polymer JR^{®}: 400 quaternierte Hydroxyethylcellulose (INCI-Bezeichnung: Polyquaternium-10) (Amerchol)

| **Aufbaucreme** | Gew.-% |
|---|---|
| Paraffinum Liquidum | 1 |
| Dehyquart F 75 | 2 |
| **Oligopeptid-Mischung^{#}** | **0,1** |
| Varisoft W 75 PG | 1,5 |
| Cetearyl Alcohol | 3,5 |
| Emulmetik 100 | 0,4 |
| Propylparaben | 0,15 |
| Cutina CP | 0,7 |
| Stearamidopropyldimethylamine | 1 |
| Dehyquart A CA | 3 |
| Citronensäure | 0,5 |
| Methylparaben | 0,15 |
| Phenoxyethanol, rein | 0,4 |
| D-Panthenol 75 % | 0,2 |
| Gluadin W 20 | 1 |
| Parfüm | 0,4 |
| Salcare SC 96 | 1 |
| Wasser, vollentsalzt | ad 100 |

- #: enthaltend - bezogen auf ihr Gewicht - mindestens 10 Gew.-% einer Mischung aus Oligopeptiden aus 8 Aminosäuren mit der Aminosäuresequenz Tyr-Glu-Glu-Glu-Ile-Arg-Val-Leu und Oligopeptiden aus 9 Aminosäuren mit der Aminosäuresequenz Leu-Tyr-Glu-Glu-Glu-Ile-Arg-Val-Leu
- Dehyquart^{®} F75: Fettalkohole-Methyltriethanolammoniummethylsulfat-dialkylester-Gemisch (INCI-Bezeichnung: Distearoylethyl Hydroxyethylmonium Methosulfate, Cetearyl Alcohol) (Henkel)
- Varisoft^{®} W 575 PG: INCI-Bezeichnung: Quaternium-87 (Goldschmidt)
- Emulmetik^{®}: 100 Lecithin (Degussa)
- Cutina^{®}: CP Cetylpalmitat (Cognis)
- Dehyquart^{®}: A-CA Trimethylhexadecylammoniumchlorid (ca. 24 -26 % Aktivsubstanz; INCI-Bezeichnung: Aqua (Water), Cetrimonium Chloride) (Cognis)
- Giuadin^{®}: W20 Weizenproteinhydrolysat (mind. 20% Festkörper; INCI-Bezeichnung: Aqua (Water), Hydrolyzed Wheat Protein, Sodium Benzoate, Phenoxyethanol, Methylparaben, Propylparaben) (Cognis)
- Salcare^{®} SC 96: ca. 50% Aktivsubstanzgehalt; INCI-Bezeichnung: Polyquaternium-37, Propylene Glycol Dicaprylate/Dicaprate, PPG-1 Trideceth-6 (CIBA)

| **Leave in Treatment** | Gew.-% |
|---|---|
| Synthalen K | 0,25 |
| Sepigel 305 | 1 |
| Dehyquart F 75 | 0,5 |
| Polymer JR 400 | 0,3 |
| Neutrol TE | 0,4 |
| **Oligopeptid-Mischung^{#}** | **0,1** |
| Parfüm | 0,3 |
| D-Panthenol 75 % | 0,5 |
| Luviskol K 30 Pulver | 0,5 |
| Gafquat 755N | 0,5 |
| Dow Corning 1401 Fluid | 1 |
| Ethanol 96 % DEP vergällt | 15 |
| Sepigel 305 | 1,6 |
| Wasser, vollentsalzt | ad 100 |

- #: enthaltend - bezogen auf ihr Gewicht - mindestens 10 Gew.-% einer Mischung aus Oligopeptiden aus 8 Aminosäuren mit der Aminosäuresequenz Tyr-Glu-Glu-Glu-Ile-Arg-Val-Leu und Oligopeptiden aus 9 Aminosäuren mit der Aminosäuresequenz Leu-Tyr-Glu-Glu-Glu-Ile-Arg-Val-Leu
- Synthalen^{®} K: Polyacrylsäure (ca. 89% Aktivsubstanzgehalt; INCI-Bezeichnung: Carbomer) (3V Sigma)
- Neutrol^{®}TE: N,N,N',N',-Tetrakis-(2-hydroxypropyl)-ethylendiamin (INCI-Bezeichnung: Tetrahydroxypropyl Ethylenediamine) (BASF)
- Luviskol^{®} K30: Polyvinylpyrrolidon (100% Festkörper; INCI-Bezeichnung: PVP) (BASF)
- Gafquat^{®} 755: Dimethylaminoethylmethacrylat-Vinylpyrrolidon-Copolymer, quaterniert mit Diethylsulfat (ca. 19% Festkörper in Wasser; INCI-Bezeichnung: Polyquaternium-11) (ISP)
- Dow Corning 1401^{®}: Dimethylcyclosiloxan Dimethylpolysiloxanol Gemisch (ca. 13% Festkörper; INCI-Bezeichnung: Cyclomethicone, Dimethiconol) (Dow Corning)
- Sepigel^{®} 305: ca. 45-49% Festkörper; INCI-Bezeichnung: Polyacryl-amide, C₁₃₋₁₄ Isoparaffin, Laureth-7) (Seppic)

| **Intensiv Kur** | Gew.-% |
|---|---|
| Varisoft W 75 PG | 1,5 |
| Paraffinum Liquidum | 1 |
| Dehyquart F 75 | 2 |
| Stearamidopropyldimethylamine | 1 |
| **Oligopeptid-Mischung^{#}** | **0,1** |
| Cetearyl Alcohol | 5 |
| Cutina GMS-V | 1 |
| Citronensäure | 0,3 |
| Dehyquart A CA | 5 |
| Salcare SC 96 | 0,5 |
| Parfüm | 0,4 |
| D-Panthenol 75 % | 0,5 |
| Wasser, vollentsalzt | ad 100 |

- #: enthaltend - bezogen auf ihr Gewicht - mindestens 10 Gew.-% einer Mischung aus Oligopeptiden aus 8 Aminosäuren mit der Aminosäuresequenz Tyr-Glu-Glu-Glu-Ile-Arg-Val-Leu und Oligopeptiden aus 9 Aminosäuren mit der Aminosäuresequenz Leu-Tyr-Glu-Glu-Glu-Ile-Arg-Val-Leu
- Cutina^{®} GMS-V: Glycerinmono/dipalmitat/stearat (INCI-Bezeichnung: Glyceryl Stearate) (Cognis)

### Wirkungsnachweis:

Durch Bestimmung des Elastizitätsmoduls mittels Zug-Dehnungsmessungen im Hookeschen Bereich wurde an Einzelhaaren untersucht, inwieweit ein Zusatz von 0,01% einer Oligopeptidmischung, die - bezogen auf ihr Gewicht - mindestens 10 Gew.-% einer Mischung aus Oligopeptiden aus 8 Aminosäuren mit der Aminosäuresequenz Tyr-Glu-Glu-Glu-Ile-Arg-Val-Leu und Oligopeptiden aus 9 Aminosäuren mit der Aminosäuresequenz Leu-Tyr-Glu-Glu-Glu-Ile-Arg-Val-Leu enthält (**entsprechend einer Einsatzkonzentration an erfindungsgemäßen Oligopeptiden von mindestens 0,001 Gew.-%, bezogen auf das Shampoo**), in einer Basisshampooformulierung einen positiven Einfluss auf die Elastizität der Haarstruktur nach 50 automatisierten Behandlungszyklen, welche neben dem Shampoonieren, dem Ausspülen und Trockenen auch eine Bestrahlung mit simuliertem Sonnenlicht der Haare beinhaltet. Als Referenz diente das unbehandelte Haar.
Für die Messungen wurde das Euro-Natur-Haar, remis, Fb. 7/0 der Firma Kerling verwendet.
Für die Messungen wurden **40** Einzelhaare jeweils in drei Stücke geteilt, zyklisch vertauscht und auf drei Messreihen verteilt.
1. Messreihe Basisshampoo
2. Messreihe Basisshampoo + 0,01% Oligopeptidmischung
3. Referenz unbehandeltes Haar

### Shampooformulierung:

Basisshampoo:
43.12 % - Natriumlaurethsulfat 25%
0.50 % - Citronensäure Monohydrat
7.00 % - Disodium Cocoamphodiacetate
0.50 % - Na-benzoat
0.20 % - Salicylsäure
48.68 % - Wasser

Applikationsmenge der Referenzlösung bzw. Testlösung 160 ml
Vorwärmen der Lösung: 5 Minuten bei 38°C
Einwirkzeit der Haare in o. g. Lösungen 3 Minuten bei 38°C
Ausspülzeit der jeweiligen Lösungen 2 Minuten.
30 Minuten Trocknen bei 80°C
60 Minuten Bestrahlen bei 765 Watt, Schwarzstandardtemperatur 50 °C, Probenraumtemperatur
37 bzw. 38°C

### Ablauf der Messungen:

1. Durchführung von 50 automatisierten Behandlungs- und Bestrahlungszyklen (mfa) für die Messreihe 1 und 2.
2. Haarquerschnittsflächenbestimmung der Messreihen 1, 2 und 3.
3. Mittels Nass-Zug-Dehnungsmessungen im Hookeschen Bereich wurde von allen Einzelhaaren der Messreihen 1, 2 und 3 das Elastizitätsmodul bestimmt.

### Messergebnisse der Elastizitätsmessungen:

| Haarquerschnittsfläche [µm²] | Elastic E-Modul [N/m²] | Elastic Gradient [N/µm²/mm] | Elastic Extension [%] |
|---|---|---|---|
| **Messreihe Nr.1:** 50 Durchläufe mit der mfa mit **Basisshampoo** | | | |
| 3319,6 | 2,08E+09 | 6,81E-05 | 2,33E-00 |
| **Messreihe Nr.2:** 50 Durchläufe mit der mfa mit **Basisshampoo + 0.01% Oligopeptidmischung** | | | |
| 3242,6 | 2,18E+09 | 7,13E-05 | 2,27E-00 |
| **Messreihe Nr.3: Unbehandeltes Haar** | | | |
| 3289,5 | 2,24E+09 | 7,38E-05 | 2,12E-05 |
| **Vergleich Messreihe Nr.1 zu Nr.2:** | | | |
| t-Test: zweiseitig für unabhängige Stichproben | | | |
| nicht unterschieden | signifikant unterschieden | signifikant unterschieden | signifikant unterschieden |
| **Vergleich Messreihe Nr.1 zu Nr.3:** | | | |
| t-Test: zweiseitig für unabhängige Stichproben | | | |
| nicht unterschieden | signifikant unterschieden | signifikant unterschieden | signifikant unterschieden |
| **Vergleich Messreihe Nr.2 zu Nr.3:** | | | |
| t-Test: zweiseitig für unabhängige Stichproben | | | |
| nicht unterschieden | nicht unterschieden | nicht unterschieden | nicht unterschieden |

### Fazit:

Mit Hilfe der 50-fachen Behandlungs- und Bestrahlungszyklen (mfa) und der anschließenden Zug-Dehnungs-Messungen konnte ein signifikanter positiver Einfluss der Oligopeptidmischung auf die Elastizität der Haarstruktur festgestellt werden. Der Zusatz von 0.01% der Oligopeptidmischung (**entsprechend einer Einsatzkonzentration an erfindungsgemäßen Oligopeptiden von mindestens 0,001 Gew.-%, bezogen auf das Shampoo**) zu einem Basisshampoo führte an den Haaren zu einer signifikanten Erhöhung des Elastizitätsmoduls. Durch diesen Zusatz verbleibt die Elastizität nach 50 Behandlungs- und Bestrahlungszyklen (mfa) auf dem Niveau von ungeschädigten Haaren.

## Patentansprüche

1. Haarbehandlungsmittel, enthaltend 0,00001 bis < 0,05 Gew.-% mindestens eines Oligopetids, das mindestens eine Aminosäuresequenz Tyr-Glu-Glu-Glu 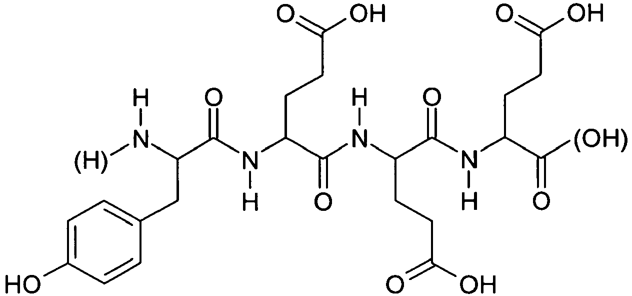 aufweist, wobei die Amino-Gruppe frei oder protoniert und die Carboxy-Gruppen frei oder deprotoniert vorliegen können,
mit der Maßgabe, daß die Gesamtmenge an Oligopeptiden, die eine Aminosäuresequenz Glu-Glu-Glu aufweisen, 0,00001 bis < 0,05 Gew.% beträgt.

2. Haarbehandlungsmittel nach Anspruch 1, **dadurch gekennzeichnet, daß** das Oligopeptid 5 bis 15 Aminosäuren, vorzugsweise 6 bis 13 Aminosäuren, besonders bevorzugt 7 bis 12 Aminosäuren und insbesondere 8, 9 oder 10 Aminosäuren umfaßt.

3. Haarbehandlungsmittel nach einem der Ansprüche 1 oder 2, **dadurch gekennzeichnet, daß** das Oligopeptid eine Molmasse von 650 bis 3000 Da, vorzugsweise von 750 bis 2500 Da, besonders bevorzugt von 850 bis 2000 Da und insbesondere von 1000 bis 1600 Da aufweist.

4. Haarbehandlungsmittel nach einem der Ansprüche 1 bis 3, **dadurch gekennzeichnet, daß** das Oligopeptid mindestens eine Aminosäuresequenz Tyr-Glu-Glu-Glu-Ile 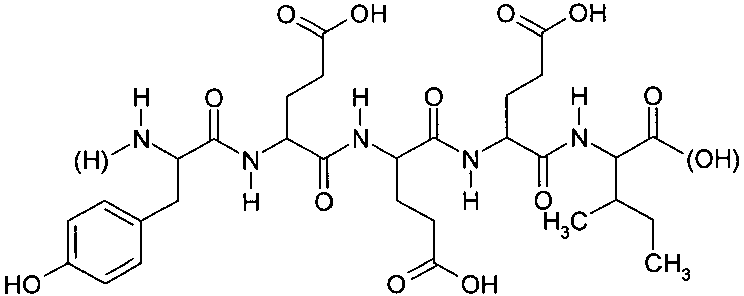 aufweist, wobei die Amino-Gruppe frei oder protoniert und die Carboxy-Gruppen frei oder deprotoniert vorliegen können.

5. Haarbehandlungsmittel nach einem der Ansprüche 1 bis 4, **dadurch gekennzeichnet, daß** das Oligopeptid mindestens eine Aminosäuresequenz Tyr-Glu-Glu-Glu-Ile-Arg 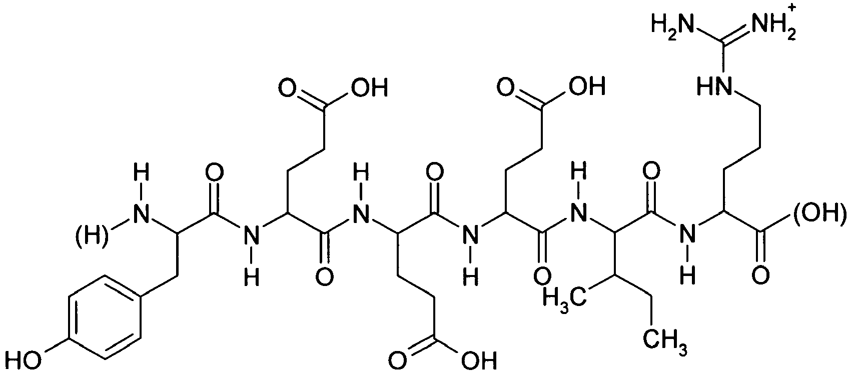 aufweist, wobei die Amino-Gruppen frei oder protoniert und die Carboxy-Gruppen frei oder deprotoniert vorliegen können.

6. Haarbehandlungsmittel nach einem der Ansprüche 1 bis 5, **dadurch gekennzeichnet, daß** das Oligopeptid mindestens eine Aminosäuresequenz Tyr-Glu-Glu-Glu-Ile-Arg-Val 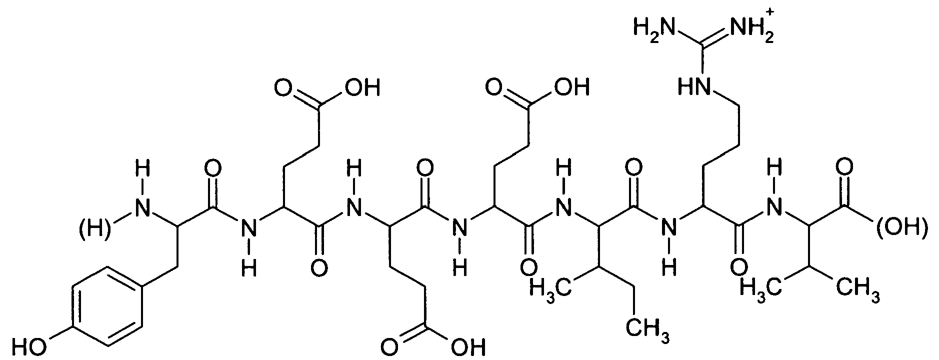 aufweist, wobei die Amino-Gruppen frei oder protoniert und die Carboxy-Gruppen frei oder deprotoniert vorliegen können.

7. Haarbehandlungsmittel nach einem der Ansprüche 1 bis 6, **dadurch gekennzeichnet, daß** das Oligopeptid mindestens eine Aminosäuresequenz Tyr-Glu-Glu-Glu-Ile-Arg-Val-Leu 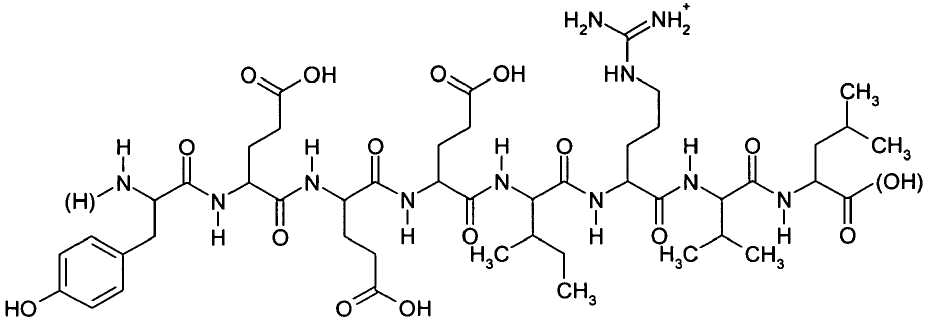 aufweist, wobei die Amino-Gruppen frei oder protoniert und die Carboxy-Gruppen frei oder deprotoniert vorliegen können.

8. Haarbehandlungsmittel nach einem der Ansprüche 1 bis 7, **dadurch gekennzeichnet, daß** das Oligopeptid mindestens eine Aminosäuresequenz Leu-Tyr-Glu-Glu-Glu-Ile-Arg-Val-Leu 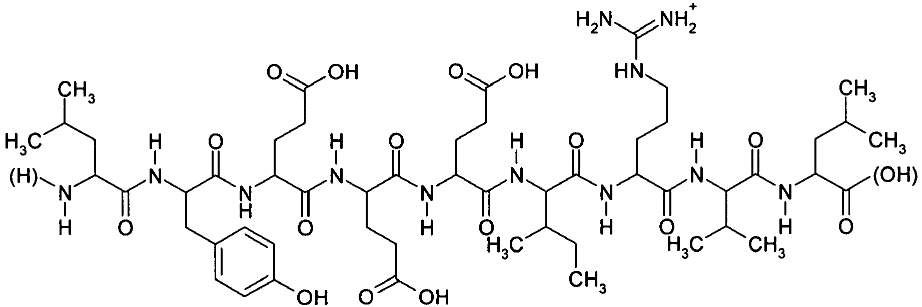 aufweist, wobei die Amino-Gruppen frei oder protoniert und die Carboxy-Gruppen frei oder deprotoniert vorliegen können.

9. Haarbehandlungsmittel nach einem der Ansprüche 1 bis 8, **dadurch gekennzeichnet, daß** es mindestens zwei voneinander verschiedene Oligopetide A und B enthält, wobei das Oligopeptid A die Aminosäuresequenz Leu-Tyr-Glu-Glu-Glu-Ile-Arg-Val-Leu 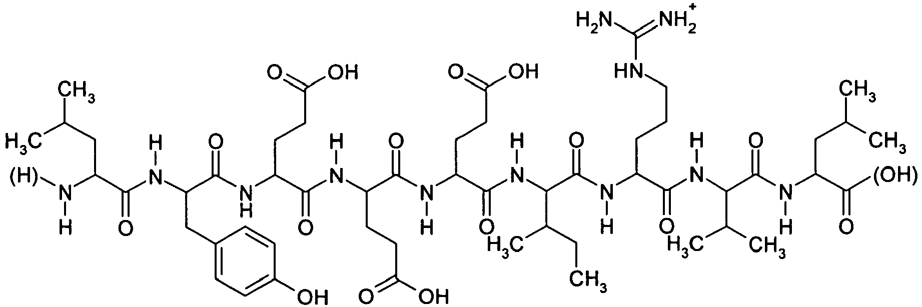 aufweist, wobei die Amino-Gruppen frei oder protoniert und die Carboxy-Gruppen frei oder deprotoniert vorliegen können
und das Oligopeptid B die Aminosäuresequenz Tyr-Glu-Glu-Glu-Ile-Arg-Val-Leu 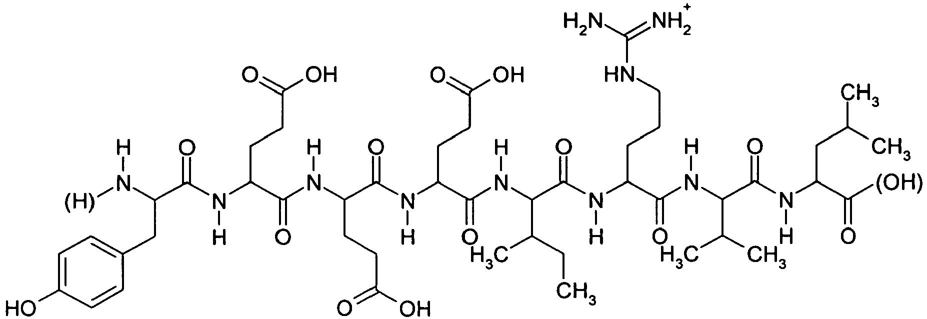 aufweist, wobei die Amino-Gruppen frei oder protoniert und die Carboxy-Gruppen frei oder deprotoniert vorliegen können,
aufweist.

10. Haarbehandlungsmittel nach einem der Ansprüche 1 bis 9, **dadurch gekennzeichnet, daß** mindestens 0,1 Gew.-%, vorzugsweise mindestens 0,5 Gew.-%, besonders bevorzugt mindestens 1 Gew.%, weiter bevorzugt mindestens 2,5 Gew.-%, noch weiter bevorzugt mindestens 5 Gew.-% und insbesondere mindestens 10 Gew.% aller im Mittel enthaltenen keratinischen Peptide die Aminosäuresequenz Tyr-Glu-Glu-Glu-Ile-Arg-Val-Leu aufweisen.

11. Haarbehandlungsmittel nach einem der Ansprüche 1 bis 10, **dadurch gekennzeichnet, daß** es - bezogen auf sein Gewicht - 0,5 bis 70 Gew.-%, vorzugsweise 1 bis 60 Gew.-% und insbesondere 5 bis 25 Gew.% anionische(s) und/oder nichtionische(s) und/oder kationische(s) und/oder amphotere(s) Tensid(e), enthält.

12. Haarbehandlungsmittel nach einem der Ansprüche 1 bis 11, **dadurch gekennzeichnet, daß** es - bezogen auf sein Gewicht - 0,05 bis 7,5 Gew.-%, vorzugsweise 0,1 bis 5 Gew.-%, besonders bevorzugt 0,2 bis 3,5 Gew.-% und insbesondere 0,25 bis 2,5 Gew.-% kationische(s) Polymer(e), enthält, wobei bevorzugte kationische(s) Polymer(e) ausgewählt ist/sind aus
- Poly(methacryloyloxyethyltrimethylammoniumchlorid) (INCI: Polyquaternium-37) und/oder;
- quaternisierten Cellulose-Derivaten (INCI: Polyquaternium 10) und/oder
- kationischen Alkylpolyglycosiden und/oder
- kationisiertem Honig und/oder
- kationischen Guar-Derivaten und/oder
- polymeren Dimethyldiallylammoniumsalzen und deren Copolymeren mit Estern und Amiden von Acrylsäure und Methacrylsäure und/oder
- Copolymeren des Vinylpyrrolidons mit quaternierten Derivaten des Dialkylaminoalkylacrylats und -methacrylats und/oder
- Vinylpyrrolidon-Vinylimidazoliummethochlorid-Copolymeren und/oder
- quaterniertem Polyvinylalkohol und/oder
- Polyquaternium 2 und/oder
- Polyquaternium-7 und/oder
- Polyquaternium 17 und/oder
- Polyquaternium 18 und/oder
- Polyquaternium 24 und/oder
- Polyquaternium 27.

13. Verwendung von Haarbehandlungsmittel nach einem der Ansprüche 1 bis 12 zur Verbesserung mindestens einer der Eigenschaften
- Zugfestigkeit von keratinischen Fasern, insbesondere menschlichen Haaren;
- Stabilisierung des Feuchtigkeitshaushaltes von keratinischen Fasern, insbesondere menschlichen Haaren;
- Kämmbarkeit von keratinischen Fasern, insbesondere menschlichen Haaren;
- Verzögerung des Alterungsprozesses von keratinischen Fasern, insbesondere menschlichen Haaren;
- Restrukturierbarkeit von keratinischen Fasern, insbesondere menschlichen Haaren, während des und nach dem Dauerwellprozeß;
- Verringerung der Elastizitätsabnahme von keratinischen Fasern, insbesondere menschlichen Haaren bei Beschädigung durch atmosphärische Einwirkungen.

## Claims

1. A hair treatment agent containing 0.00001 to < 0.05 wt.% of at least one oligopeptide having at least one amino acid sequence Tyr-Glu-Glu-Glu 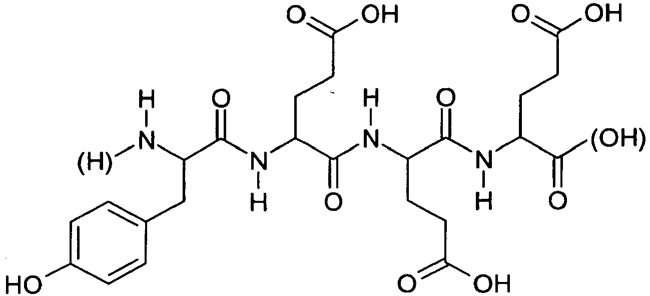 wherein the amino group may be in free or protonated form and the carboxy groups may be in free or deprotonated form,
with the proviso that the total amount of oligopeptides having an amino acid sequence Glu-Glu-Glu is less than 0.00001 to < 0.05 wt.%.

2. The hair treatment agent according to claim 1, **characterized in that** the oligopeptide comprises from 5 to 15 amino acids, preferably from 6 to 13 amino acids, particularly preferably from 7 to 12 amino acids and in particular 8, 9 or 10 amino acids.

3. The hair treatment agent according to one of claims 1 or 2, **characterized in that** the oligopeptide has a molar mass of from 650 to 3000 Da, preferably from 750 to 2500 Da, particularly preferably from 850 to 2000 Da and in particular from 1000 to 1600 Da.

4. The hair treatment agent according to one of claims 1 to 3, **characterized in that** the oligopeptide has at least one amino acid sequence Tyr-Glu-Glu-Glu-Ile 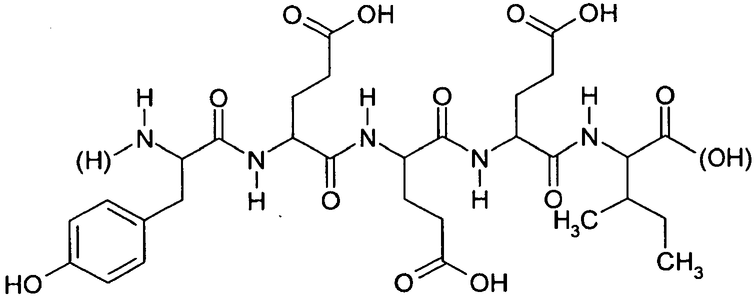 wherein the amino group may be in free or protonated form and the carboxy groups may be in free or deprotonated form.

5. The hair treatment agent according to one of claims 1 to 4, **characterized in that** the oligopeptide has at least one amino acid sequence Tyr-Glu-Glu-Glu-Ile-Arg 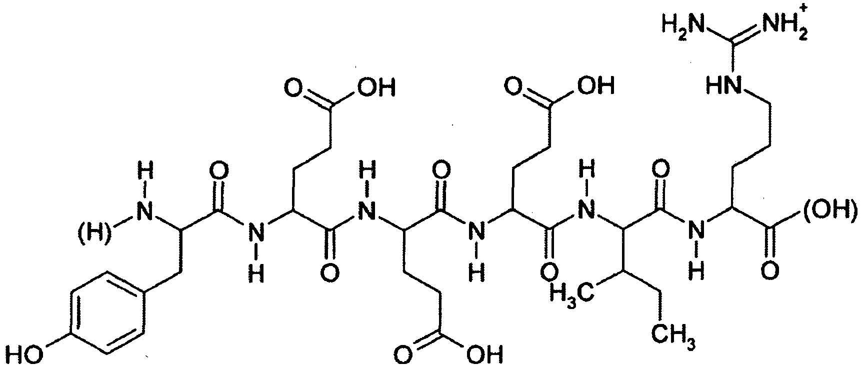 wherein the amino groups may be in free or protonated form and the carboxy groups may be in free or deprotonated form.

6. The hair treatment agent according to one of claims 1 to 5, **characterized in that** the oligopeptide has at least one amino acid sequence Tyr-Glu-Glu-Glu-Ile-Arg-Val 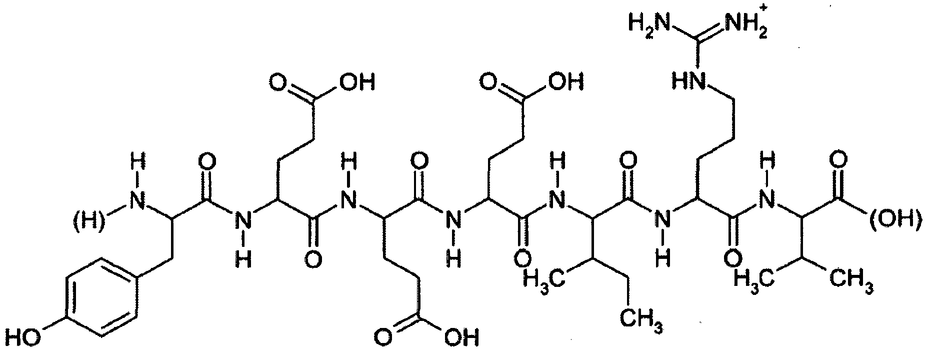 wherein the amino groups may be in free or protonated form and the carboxy groups may be in free or deprotonated form.

7. The hair treatment agent according to one of claims 1 to 6, **characterized in that** the oligopeptide has at least one amino acid sequence Tyr-Glu-Glu-Glu-Ile-Arg-Val-Leu 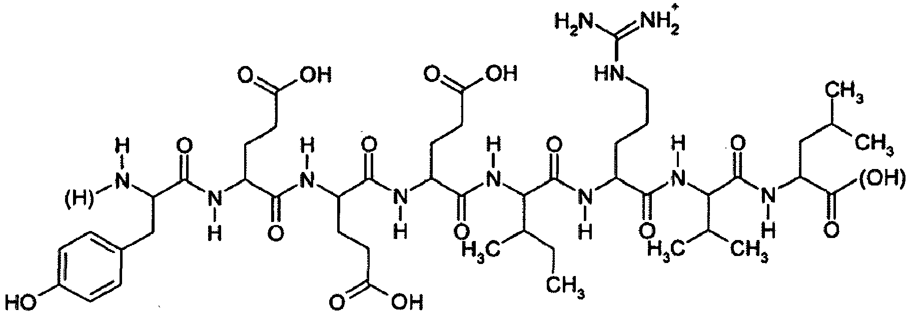 wherein the amino groups may be in free or protonated form and the carboxy groups may be in free or deprotonated form.

8. The hair treatment agent according to one of claims 1 to 7, **characterized in that** the oligopeptide has at least one amino acid sequence Leu-Tyr-Glu-Glu-Glu-Ile-Arg-Val-Leu 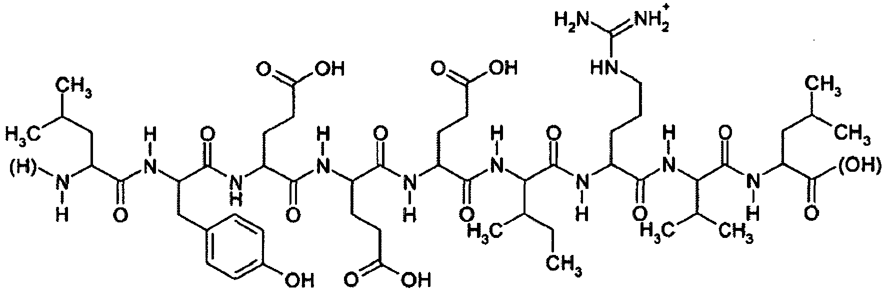 wherein the amino groups may be in free or protonated form and the carboxy groups may be in free or deprotonated form.

9. The hair treatment agent according to one of claims 1 to 8, **characterized in that** it contains at least two oligopeptides A and B that are different from one another, the oligopeptide A having the amino acid sequence Leu-Tyr-Glu-Glu-Glu-Ile-Arg-Val-Leu, 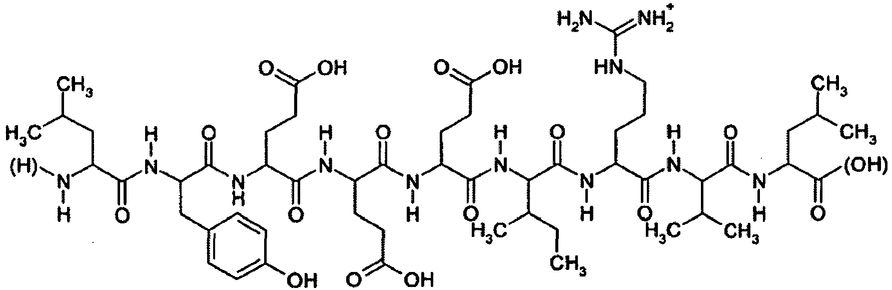 wherein the amino groups may be in free or protonated form and the carboxy groups may be in free or deprotonated form,
and the oligopeptide B having the amino acid sequence Tyr-Glu-Glu-Glu-Ile-Arg-Val-Leu 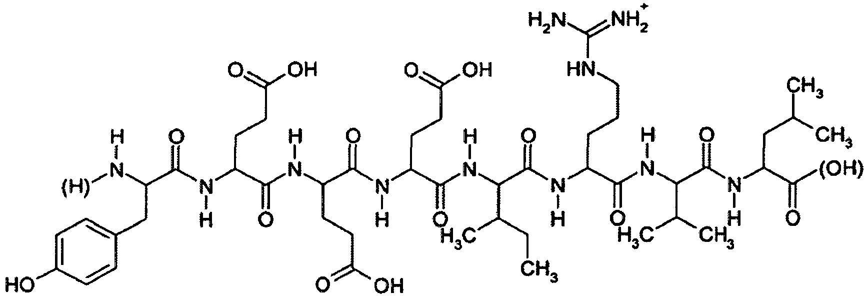 wherein the amino groups may be in free or protonated form and the carboxy groups may be in free or deprotonated form.

10. The hair treatment agent according to one of claims 1 to 9, **characterized in that** at least 0.1 wt.%, preferably at least 0.5 wt.%, particularly preferably at least 1 wt.%, more preferably at least 2.5 wt.%, even more preferably at least 5 wt.% and in particular at least 10 wt.% of all the keratin peptides contained in the agent have the amino acid sequence Tyr-Glu-Glu-Glu-Ile-Arg-Val-Leu.

11. The hair treatment agent according to one of claims 1 to 10, **characterized in that** it contains, based on its weight, from 0.5 to 70 wt.%, preferably from 1 to 60 wt.% and in particular from 5 to 25 wt.% of one or more anionic and/or non-ionic and/or cationic and/or amphoteric surfactant(s).

12. The hair treatment agent according to one of claims 1 to 11, **characterized in that** it contains, based on its weight, from 0.05 to 7.5 wt.%, preferably from 0.1 to 5 wt.%, particularly preferably from 0.2 to 3.5 wt.% and in particular 0.25 to 2.5 wt.% of one or more cationic polymer(s), preferred cationic polymer(s) being selected from
- poly(methacryloyloxyethyl trimethylammonium chloride) (INCI: Polyquaternium-37), and/or;
- quaternized cellulose derivatives (INCI: Polyquaternium 10), and/or
- cationic alkyl polyglycosides, and/or
- cationized honey, and/or
- cationic guar derivatives, and/or
- polymeric dimethyl diallyl ammonium salts and copolymers thereof with esters and amides of acrylic acid and methacrylic acid, and/or
- copolymers of vinyl pyrrolidone with quaternized derivatives of dialkylaminoalkyl acrylate and methacrylate, and/or
- vinylpyrrolidone-vinylimidazolium methochloride copolymers, and/or
- quaternized polyvinyl alcohol, and/or
- Polyquaternium 2, and/or
- Polyquaternium-7, and/or
- Polyquaternium 17, and/or
- Polyquaternium 18, and/or
- Polyquaternium 24, and/or
- Polyquaternium 27.

13. The use of hair treatment agents according to one of claims 1 to 12 for improving at least one of the following properties:
- tensile strength of keratinous fibers, in particular of human hair;
- stabilizing the moisture balance of keratinous fibers, in particular of human hair;
- combability of keratinous fibers, in particular of human hair;
- delaying the aging process of keratinous fibers, in particular of human hair;
- restructurability of keratinous fibers, in particular of human hair, during and after the perming process;
- reducing the loss of elasticity in keratinous fibers, in particular in human hair, when damaged by atmospheric influences.

## Revendications

1. Agent de traitement capillaire contenant 0,00001 à moins de 0,05% en poids d'au moins un oligopeptide qui comporte au moins une séquence d'acides aminés Tyr-Glu-Glu-Glu 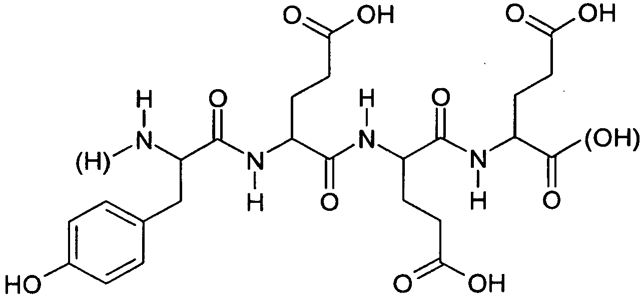 le groupe amino pouvant se présenter sous forme libre ou protonée et les groupes carboxyle pouvant se présenter sous forme libre ou déprotonée,
à condition que la quantité totale d'oligopeptides, qui comportent une séquence d'acides aminés Glu-Glu-Glu, soit de 0,00001 à moins de 0,05% en poids.

2. Agent de traitement capillaire selon la revendication 1, **caractérisé en ce que** l'oligopeptide comporte 5 à 15 acides aminés, de préférence 6 à 13 acides aminé, de manière particulièrement préférée 7 à 12 acides aminés et en particulier 8, 9 ou 10 acides aminés.

3. Agent de traitement capillaire selon l'une des revendications 1 ou 2, **caractérisé en ce que** l'Oligopeptide a un poids moléculaire de 650 à 3000 Da, de préférence de 750 à 2500 Da, de manière particulièrement préférée de 850 à 2000 Da et en particulier de 1000 à 1600 Da.

4. Agent de traitement capillaire selon l'une des revendications 1 à 3, **caractérisé en ce que** l'oligopeptide comporte au moins une séquence d'acide aminé Tyr-Glu-Glu-Glu-Ile 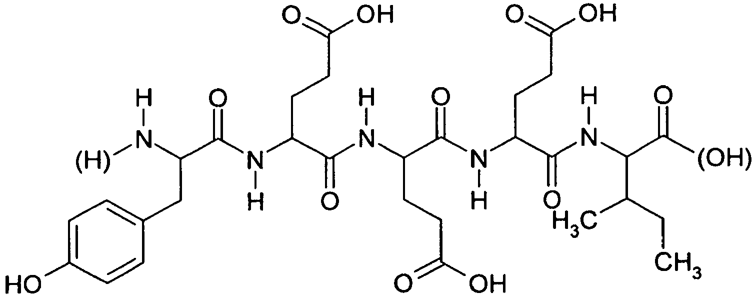 le groupe amino pouvant se présenter sous forme libre ou protonée et les groupes carboxyle pouvant se présenter sous forme libre ou déprotonée.

5. Agent de traitement capillaire selon l'une des revendications 1 à 4, **caractérisé en ce que** l'oligopeptide comporte au moins une séquence d'acides aminés Tyr-Glu-Glu-Glu-Ile-Arg 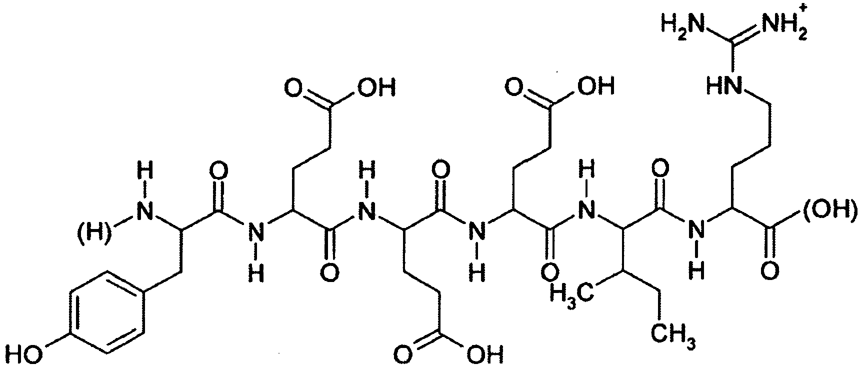 le groupe amino pouvant se présenter sous forme libre ou protonée et les groupes carboxyle pouvant se présenter sous forme libre ou déprotonée.

6. Agent de traitement capillaire selon l'une des revendications 1 à 5, **caractérisé en ce que** l'oligopeptide comporte au moins une séquence d'acides aminés Tyr-Glu-Glu-Glu-Ile-Arg-Val 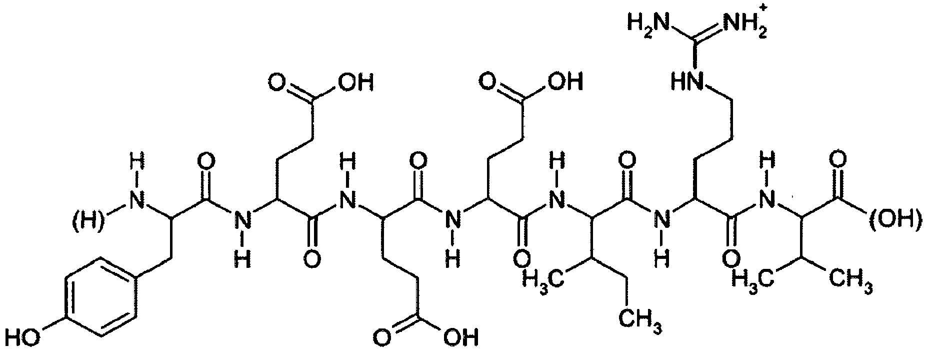 le groupe amino pouvant se présenter sous forme libre ou protonée et les groupes carboxyle pouvant se présenter sous forme libre ou déprotonée.

7. Agent de traitement capillaire selon l'une des revendications 1 à 6, **caractérisé en ce que** l'oligopeptide comporte au moins une séquence d'acides aminés Tyr-Glu-Glu-Glu-Ile-Arg-Val-Leu 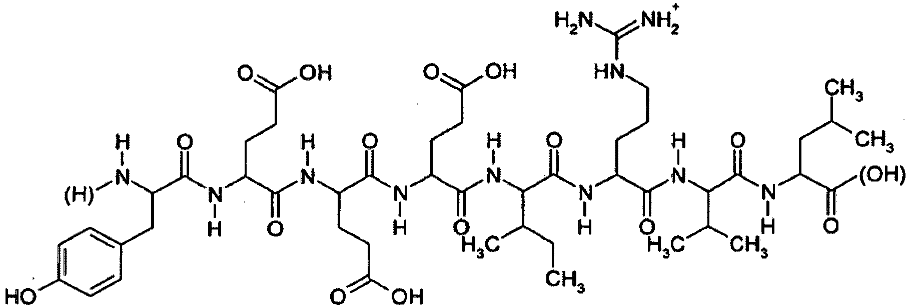 le groupe amino pouvant se présenter sous forme libre ou protonée et les groupes carboxyle pouvant se présenter sous forme libre ou déprotonée.

8. Agent de traitement capillaire selon l'une des revendications 1 à 7, **caractérisé en ce que** l'oligopeptide comporte au moins une séquence d'acides aminés Leu-Tyr-Glu-Glu-Glu-Ile-Arg-Val-Leu 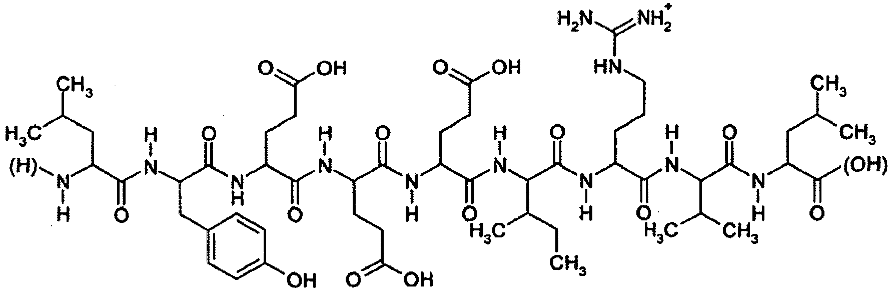 le groupe amino pouvant se présenter sous forme libre ou protonée et les groupes carboxyle pouvant se présenter sous forme libre ou déprotonée.

9. Agent de traitement capillaire selon l'une des revendications 1 à 8, **caractérisé en ce qu'**il contient au moins deux oligopeptides A et B différents l'un de l'autre, l'oligopeptide A comportant la séquence d'acides aminés Leu-Tyr-Glu-Glu-Glu-Ile-Arg-Val-Leu 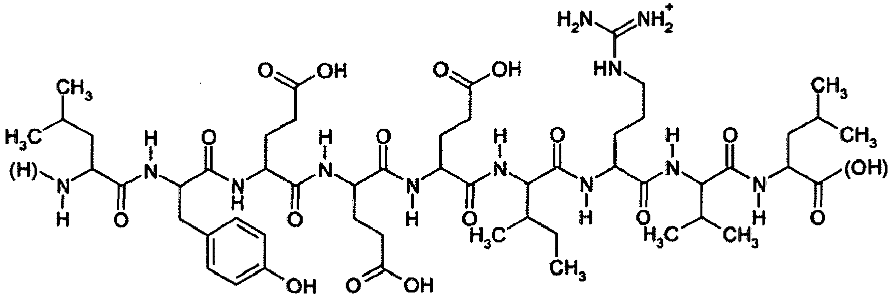 le groupe amino pouvant se présenter sous forme libre ou protonée et les groupes carboxyle pouvant se présenter sous forme libre ou déprotonée,
et l'oligopeptide B la séquence d'acides aminés Tyr-Glu-Glu-Glu-Ile-Arg-Val-Leu 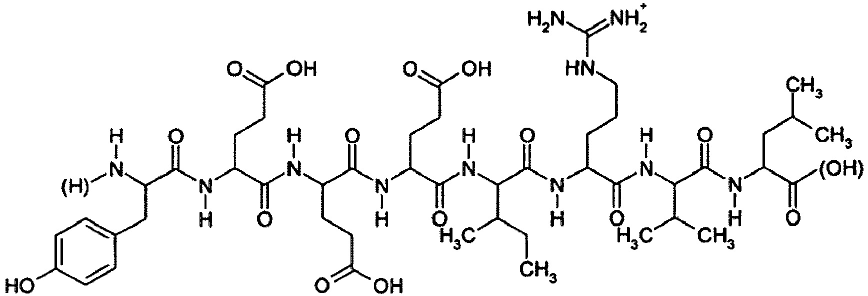 le groupe amino pouvant se présenter sous forme libre ou protonée et les groupes carboxyle pouvant se présenter sous forme libre ou déprotonée.

10. Agent de traitement capillaire selon l'une des revendications 1 à 9, **caractérisé en ce qu'**au moins 0,1 % en poids, de préférence au moins 0,5% en poids, de manière particulièrement préférée au moins 1% en poids, plus préférablement au moins 2,5% en poids, encore plus préférablement au moins 5% en poids et en particulier 10% en poids, de tous les peptides de kératine contenus dans l'agent ont la séquence d'acides aminés Tyr-Glu-Glu-Glu-Ile-Arg-Val-Leu.

11. Agent de traitement capillaire selon l'une des revendications 1 à 10, **caractérisé en ce qu'**il contient, sur la base de son poids, 0,5 à 70% en poids, de préférence 1 à 60% en poids et en particulier 5 à 25% en poids de tensioactif(s) anionique(s) et/ou non-ionique(s) et/ou cationique(s) et/ou amphotère(s).

12. Agent de traitement capillaire selon l'une des revendications 1 à 11, **caractérisé en ce qu'**il contient, sur la base de son poids, 0,05 à 7,5% en poids, de préférence 0,1 à 5% en poids, de manière particulièrement préférée 0,2 à 3,5% en poids et en particulier de 0,25 à 2,5% en poids de polymère(s) cationique(s), le ou les polymères cationiques préférés étant sélectionnés parmi
- le poly(chlorure de méthacryloyloxyéthyltriméthylammonium) (INCI: Polyquaternium-37) et/ou ;
- les dérivés quaternisés de la cellulose (INCI : Polyquaternium 10) et/ou
- les polyglycosides d'alkyle cationiques et/ou
- le miel cationisé et/ou
- les dérivés de guar cationiques et/ou
- les sels de diméthyldiallylammonium polymères et leurs copolymères avec des esters et des amides de l'acide acrylique et de l'acide méthacrylique et/ou
- les copolymères de vinylpyrrolidone avec des dérivés quaternisés de l'acrylate et du méthacrylate de dialkylaminoalkyle et/ou
- les copolymères de vinylpyrrolidone-méthpchlorure de vinylimidazolium et/ou
- l'alcool polyvinylique quaternisé et/ou
- le Polyquaternium 2, et/ou
- le Polyquaternium-7 et/ou
- le Polyquaternium 17 et/ou
- le Polyquaternium 18 et/ou
- le Polyquaternium 24 et/ou
- le Polyquaternium 27

13. Utilisation d'agent de traitement capillaire selon l'une des revendications 1 à 12 pour améliorer au moins une des propriétés suivantes
- résistance à la traction de fibres de kératine, en particulier de cheveux humains ;
- stabilisation de la teneur en humidité de fibres de kératine, en particulier de cheveux humains ;
- aptitude au peignage de fibres de kératine, en particulier de cheveux humains ;
- retard du processus de vieillissement de fibres de kératine, en particulier de cheveux humains ;
- capacité de restructuration de fibres de kératine, en particulier de cheveux humains, pendant et après la réalisation d'une permanente ;
- réduction de la perte d'élasticité de fibres de kératine, en particulier de cheveux humains, due à des influences atmosphériques.
